# EUROPEAN PATENT APPLICATION

(11) **EP 4 793 368 A1**
(43) Date of publication of application: **19.08.2026**
(21) Application number: 24938360.5
(22) Date of filing: 05.06.2024
(51) Int. Cl.: C12Q 1/6883, G01N 33/68, G01N 21/64, G01N 15/14, C07K 14/705

(54) **MARKER FOR DIAGNOSING SYSTEMIC INFLAMMATION, AND USE**

(30) Priority: 17.05.2024 CN 202410614757; 17.05.2024 CN 202410614751
(71) Applicant: Hangzhou Enryco Biotechnology Co., Ltd., Hangzhou, Zhejiang 310000 (CN)
(72) Inventor: MA, Yilei, Hangzhou, Zhejiang 310016 (CN)
(74) Representative: Dolphin, Kirsty Mairi
(86) International application number: PCT/CN2024/097452
(87) International publication number: WO 2025/236340

(57) **Abstract**

The present invention provides a biomarker and its application for diagnosing systemic inflammation. The biomarker includes ADGRE3 mRNA or ADGRE3 protein, or fragments of ADGRE3 mRNA or ADGRE3 protein. The biomarker shows significantly different levels in samples from patients with systemic inflammation compared to healthy individuals. Clinical validation has confirmed the high sensitivity and specificity of this biomarker for diagnosis. Therefore, detecting the transcription level of ADGRE3 mRNA or the expression level of ADGRE3 protein in an individual sample can accurately diagnose systemic inflammation. Compared with existing inflammatory biomarkers, ADGRE3 mRNA and ADGRE3 protein exhibit higher sensitivity and specificity in diagnosing systemic inflammation.

## Description

### CROSS-REFERENCE TO RELATED APPLICATIONS

This application claims priority to Chinese Patent Applications Nos. 202410614757.3, filed on May 17, 2024, and 202410614751.6, filed on May 17, 2024. The entire disclosures of the specifications, claims, abstracts, and drawings of the applications are incorporated herein by reference in their entirety.

### TECHNICAL FIELD

The present invention relates to diagnostic reagents and methods for diseases, and more particularly, to a biomarker capable of diagnosing systemic inflammation and its application.

### BACKGROUND OF THE INVENTION

The following background is provided for informational purposes only and does not limit the scope of the invention in any way.

Systemic inflammation refers to a non-specific inflammatory response involving the entire body, typically triggered by infections, autoimmune diseases, injuries, or other pathological conditions. Such inflammatory responses may lead to multi-organ dysfunction and, in severe cases, may become life-threatening. Therefore, timely and accurate diagnosis of systemic inflammation is crucial for guiding treatment and improving patient prognosis.

In recent years, biomarkers have played a critical role in the diagnosis of systemic inflammation, offering important information about the inflammatory state, disease severity, and therapeutic responses.

Systemic inflammation is a protective response involving immune cells, blood vessels, and various molecular mediators. During systemic inflammation, stimuli activate immune cells such as monocytes/macrophages, neutrophils, and lymphocytes, leading to the release of large quantities of cytokines (e.g., IL-6, IL-1β, TNF-α, IFN-γ), which regulate the synthesis of acute phase reactants (APRs), thus altering their plasma concentrations. These cytokines and APR changes reflect the body's response to inflammation and are used as diagnostic and monitoring indicators.

C-reactive protein (CRP), a liver-derived APR, is one of the most widely used clinical indicators of inflammation. It begins to rise 6-8 hours after the onset of acute inflammation, with peak levels that can be several hundred times higher than normal. Levels return to baseline upon resolution of the condition. The extent of CRP elevation correlates with the severity of inflammation and can signal complications or therapeutic effectiveness.

Interleukin-6 (IL-6), a key component of the cytokine network induced by IL-1 and TNF-α, is a primary mediator of the acute-phase response and induces the production of CRP, SAA, and PCT. IL-6 elevation is closely associated with inflammation progression, especially in sepsis and other systemic infections.

Tumor necrosis factor-α (TNF-α) plays a pivotal role in initiating cytokine storm responses, and its rapid elevation reflects acute-phase inflammation. Anti-TNF-α agents have been successfully used to treat inflammatory diseases such as rheumatoid arthritis, ankylosing spondylitis, and inflammatory bowel disease.

Erythrocyte sedimentation rate (ESR), a traditional inflammation marker, is often increased due to elevated fibrinogen levels. Although its specificity is low, ESR still has diagnostic value as a supplementary reference.

Procalcitonin (PCT) is another important inflammation marker, especially elevated in systemic inflammation caused by bacterial infections. Measuring PCT helps distinguish between bacterial and viral infections and has significant clinical utility.

While the above markers (CRP, IL-6, TNF-α, ESR, and PCT) help assess inflammation severity and monitor disease progression or treatment response, they generally lack specificity for systemic vs. localized inflammation. Differences between patients with systemic inflammation and healthy individuals are not always significant, resulting in low sensitivity. Often, multiple indicators and imaging are needed for accurate diagnosis.

Multiple viral infections (e.g., influenza virus, SARS-CoV-2, dengue virus, RSV, cytomegalovirus, and EBV) can cause systemic inflammatory responses. During such infections, the immune system releases various inflammatory mediators to activate immune cells, promoting viral clearance and tissue repair. However, conventional markers such as PCT, HBP, and nCD64 show little change in viral infections, and CRP has low sensitivity. Therefore, effective biomarkers are lacking for diagnosing systemic inflammation caused by viral infections and for monitoring inflammation severity.

Unlike localized inflammation, systemic inflammation is more severe and complex to diagnose. Accurate and timely diagnosis is critical for treatment decisions and prognosis. Therefore, there is a clinical need for a highly sensitive and specific biomarker to diagnose systemic inflammation.

### SUMMARY OF THE INVENTION

To address the limitations of the prior art, the present invention provides a biomarker for diagnosing systemic inflammation and uses thereof. The biomarker includes ADGRE3 mRNA or fragments thereof, and/or an ADGRE3 protein or a fragment thereof. Changes in the level or concentration of this biomarker can be used to diagnose whether an individual has systemic inflammation, monitor disease severity, predict disease progression, and guide specific treatment strategies.

The inventors discovered that the expression levels of this biomarker in neutrophils differs significantly between individuals with systemic inflammation and healthy individuals. Clinical validation confirmed its diagnostic utility. Thus, detection of ADGRE3 mRNA or protein in an individual sample allows for diagnosing whether an individual is suffering from systemic inflammation, monitoring disease progression, and evaluating therapeutic response. When combined with other traditional markers, it can also help differentiate between bacterial and viral causes of systemic inflammation.

Compared with conventional inflammatory biomarkers, ADGRE3 mRNA and/or protein showed improved diagnostic performance in the tested cohorts.

High sensitivity refers to: First, among the same cohort of patients with systemic inflammation, significance analysis shows that the ADGRE3 biomarker (ADGRE3 mRNA or ADGRE3 protein) exhibits an extremely significant decrease, whereas other traditional inflammatory markers show only small changes. Alternatively, although other traditional inflammatory markers can diagnose systemic inflammation, they show little or no significant difference between healthy individuals and those with local inflammation. In contrast, the markers of the present invention display a significant or extremely significant difference in levels between samples from healthy individuals, patients with local inflammation, and patients with systemic inflammation.

High Specificity refers to: First, in a case cohort encompassing healthy subjects (without inflammation), patients with non-systemic inflammation (patients with local inflammation or non-inflammatory diseases), and patients with systemic inflammation, the ADGRE3 indicator is capable of excluding patients with non-systemic inflammation, including those with local inflammation, which cannot be achieved by existing inflammatory indicators. Second, among patients with systemic inflammation, the ADGRE3 indicator also enables specific differentiation between different types of infectious systemic inflammation; for example, it can distinguish bacterial inflammation from non-bacterial inflammation, thereby assisting clinical treatment. Although the marker of the present invention needs to be combined with traditional bacterial markers when differentiation between bacterial infection and viral infection is required, this does not negate the role and function of the marker of the present invention in the above diagnoses. Furthermore, significant differences also exist between different types of systemic inflammation in patients with systemic inflammation. For instance, a significant difference is observed between patients with sepsis and those with non-infectious systemic inflammation. Therefore, it can be understood that the marker can be used to distinguish sepsis from non-infectious systemic inflammation.

Therefore, one aspect of the present invention provides the use of ADGRE3 mRNA or a fragment thereof, or ADGRE3 protein or a fragment thereof, or a corresponding amino acid sequence or peptide fragment, in the preparation of diagnostic reagent for determining whether an individual has systemic inflammation.

In the present invention, the detection of ADGRE3 mRNA or an ADGRE3 mRNA fragment, as well as the detection of ADGRE3 protein or an ADGRE3 protein fragment - that is, the detection of the level or content of ADGRE3 mRNA and the level or content of ADGRE3 protein - all refer to the determination of the content of ADGRE3 mRNA or an ADGRE3 mRNA fragment and ADGRE3 protein or an ADGRE3 protein fragment in a sample. In some embodiments, the level of ADGRE3 mRNA is determined by the copy number obtained by PCR amplification, and the expression level of ADGRE3 protein is determined by the mean fluorescence intensity (MFI) measured by flow cytometry. In some embodiments, threshold values are set for the copy number of ADGRE3 mRNA and the MFI value of ADGRE3 protein to classify samples, so as to diagnose or distinguish systemic inflammation and related diseases. It will be appreciated that any other method capable of characterizing the content of ADGRE3 protein or mRNA in a sample may be used; for example, concentration content, relative concentration content, and comparison with normal levels in samples from healthy individuals may all be employed for the assay.

In some embodiments, the present invention employs flow cytometry to detect the content of ADGRE3 protein in neutrophils in blood samples from healthy non-inflammatory subjects. The mean fluorescence intensity (MFI) of ADGRE3 protein in each sample is calculated to obtain an average MFI value of ADGRE3 protein in healthy subjects. Under identical conditions, the MFI of ADGRE3 protein in neutrophils in patient samples is determined. A neutrophil ADGRE3 expression index (nADGRE3) for the patient is then obtained by dividing the MFI of ADGRE3 protein in patient neutrophils by the average MFI of ADGRE3 protein in healthy controls: nADGRE3 = MFI of ADGRE3 protein in patient neutrophils / MFI of ADGRE3 protein in healthy control neutrophils. Samples are classified by comparing nADGRE3 with a predetermined threshold, thereby enabling diagnosis or differentiation of systemic inflammation and related diseases.

Although nADGRE3 is one method for thresholding, any established method combining ADGRE3 mRNA or protein with known classification techniques can achieve the same diagnostic outcome and is within the scope of the invention.

In some embodiments, white blood cells include lymphocytes, neutrophils, monocytes, eosinophils, and basophils. Among these cell types, neutrophils highly express ADGRE3 protein, while monocytes express ADGRE3 protein at lower levels. Therefore, a sample with neutrophils is primarily used for protein detection. Any detection method may be used to independently detect the protein in neutrophils. Monocytes also express ADGRE3 protein, and its expression level correlates with that in neutrophils, and thus also correlates with the occurrence of systemic inflammation. Therefore, monocytes may also be selected as the sample source. The distribution of mRNA is similar to that of the protein; however, mRNA detection cannot be resolved specifically to neutrophils or monocytes. For this reason, the detection target is generally total leukocytes. Nevertheless, this does not exclude embodiments in which the ADGRE3 mRNA level is detected in any one type of leukocyte, including lymphocytes, neutrophils, monocytes, eosinophils, and basophils.

The sample in the present invention is not limited to neutrophils, monocytes, or leukocytes, provided that it contains ADGRE3 mRNA, ADGRE3 protein, or a fragment thereof derived directly or indirectly from such cells. Detection of the transcription level of ADGRE3 mRNA in leukocytes, or the level of ADGRE3 protein in neutrophils and monocytes in a test sample only specifies that the ADGRE3 mRNA or ADGRE3 protein is derived from leukocytes, neutrophils, or monocytes. However, ADGRE3 mRNA or ADGRE3 protein is not necessarily located exclusively in neutrophils, monocytes or leukocytes; it may also be present in extracellular physiological environments such as blood and tissue fluid. The content of ADGRE3 mRNA or ADGRE3 protein in such physiological environments can also be used for the diagnosis of systemic inflammation. Alternatively, samples obtained by rupturing neutrophils, monocytes, or leukocytes, or by other methods for extracting intracellular substances, also contain ADGRE3 mRNA and fragments thereof, as well as ADGRE3 protein and fragments thereof. Such samples may also be used as diagnostic samples for systemic inflammation.

Fragments of ADGRE3 mRNA or protein refer to any naturally occurring fragments present in the individual.

Taking ADGRE3 protein as an example, ADGRE3 is a membrane protein having intracellular domain, transmembrane domain, and extracellular domain.The intracellular domain means that part of the protein is located inside the cell membrane, the extracellular domain means that part of the protein is located outside the cell membrane, and the transmembrane domain means that one part of the protein is located inside the cell membrane and another part is located outside the cell membrane. Therefore, the content of ADGRE3 protein can be determined by detecting the extracellular, intracellular, and transmembrane regions of the transmembrane protein (i.e., fragments of ADGRE3 protein). Of course, in some embodiments, cells can be lysed to extract membrane proteins before measuring the protein content. In other embodiments, ADGRE3 protein can be enzymatically cleaved into various fragments under the action of various enzymes in vivo or in vitro, and detection of the content of these fragments or any one fragment can likewise determine the content of ADGRE3 protein, thereby enabling diagnosis of systemic inflammation.

The level of ADGRE3 mRNA or ADGRE3 protein in a sample serves a diagnostic function for systemic inflammation, and fragments of the mRNA or protein are homologous to the full-length mRNA nucleotide sequence and full-length protein amino acid sequence. Therefore, in principle, detecting fragments of ADGRE3 mRNA or ADGRE3 protein can also diagnose systemic inflammation based on their measured levels.

In some embodiments, the reagent for diagnosing systemic inflammation is used to detect ADGRE3 mRNA or fragments thereof in leukocytes of an individual.

In some embodiments, the reagent for diagnosing systemic inflammation is used to detect ADGRE3 mRNA or fragments thereof in a sample obtained from the individual, wherein the sample includes one or more of peripheral blood, urine, secretions, pus, body fluids, cerebrospinal fluid, or fresh tissue.

Leukocytes are present in various tissues and body fluids of the human body, with a predominant distribution in peripheral blood in healthy individuals. Urine, secretions, pus, body fluids, cerebrospinal fluid, and fresh tissues also contain leukocytes. In patients with systemic inflammation, leukocytes infiltrate inflamed tissues. Therefore, leukocytes can be detected in various clinical sample types. Any sample containing leukocytes or substances derived from leukocytes (referred to herein as intracellular leukocyte components) can be used as a sample for detecting ADGRE3 mRNA or fragments thereof to diagnose systemic inflammation.

In some embodiments, the systemic inflammation comprises infectious or non-infectious systemic inflammation.

In some embodiments, the infectious systemic inflammation is caused by one or more of bacterial infection, viral infection, fungal infection, or parasitic infection.

In some embodiments, the viral infections include one or more of influenza virus, dengue virus, SARS-CoV-2 (novel coronavirus), respiratory syncytial virus (RSV), cytomegalovirus (CMV), Epstein-Barr virus (EBV), or SFTSV.

The above viral infections are representative causes of systemic inflammation. In addition to the systemic inflammation caused by influenza virus, dengue virus, SARS-CoV-2, RSV, CMV, EBV, or SFTSV, systemic inflammation caused by other viral infections can also be diagnosed by detecting ADGRE3 mRNA or fragments thereof, or ADGRE3 protein or fragments thereof, as disclosed in the present invention.

In some embodiments, the non-infectious systemic inflammation includes one or more of autoimmune diseases, transplant rejection, inflammatory bowel disease, allergic reactions, metabolic disorders, or inflammation caused by tissue injury.

In some embodiments, the autoimmune diseases include one or more of systemic lupus erythematosus, rheumatoid arthritis, vasculitis, or ankylosing spondylitis.

In some embodiments, the transplant rejection-induced inflammation includes inflammation caused by bone marrow, kidney, or liver transplantation.

In addition to inflammation (i.e., systemic inflammation) caused by bone marrow, kidney, or liver transplantation, inflammation due to rejection of other transplanted organs may also be diagnosed by detecting ADGRE3 mRNA or fragments thereof, or ADGRE3 protein or fragments thereof, as disclosed in the present invention.

In some embodiments, the systemic inflammation comprises sepsis.

In some embodiments, the sepsis is caused by one or more infections of the lungs, biliary tract, urinary tract, digestive tract, bloodstream, reproductive tract, abdominal cavity, central nervous system, skin and soft tissues, or by pyogenic osteomyelitis.

In addition to the above-mentioned infection sites, sepsis arising from any other foci may also be diagnosed by detecting ADGRE3 mRNA or fragments thereof, or ADGRE3 protein or fragments thereof, as disclosed in the present invention.

Second aspect of the invention: The present invention provides a biomarker for use in the preparation of a reagent for diagnosing whether an individual has systemic inflammation caused by bacterial or viral infection, wherein the biomarker comprises ADGRE3 mRNA or fragments thereof; or ADGRE3 protein or fragments thereof.

In some embodiments, the biomarker is used to prepare a reagent for differential diagnosis between Gram-positive bacterial infection-induced systemic inflammation and H1N1 virus infection-induced systemic inflammation, wherein the biomarker comprises ADGRE3 mRNA or fragments thereof; or ADGRE3 protein or fragments thereof.

Since Gram-positive bacterial infections and H1N1 virus infections may present with similar clinical symptoms, effective treatment of systemic inflammation requires accurate determination of the cause. The use of ADGRE3 mRNA or protein enables distinction between Gram-positive bacterial and H1N1 viral infections, allowing for targeted therapeutic interventions.

In some embodiments, the biomarker is used to prepare a reagent for diagnosing whether an individual has dengue virus infection-induced systemic inflammation.

In some embodiments, the biomarker is used to prepare a reagent for diagnosing whether an individual has COVID-19 (SARS-CoV-2) infection-induced systemic inflammation.

Further, the diagnosis of COVID-19 infection-induced systemic inflammation includes the diagnosis of asymptomatic cases or symptomatic cases of systemic inflammation caused by COVID-19 infection.

In some embodiments, a subset of individuals infected with COVID-19 are asymptomatic and do not exhibit clinical symptoms, making them difficult to detect clinically. However, systemic inflammation may still occur in these individuals or may be aggravated by COVID-19 infection. According to publicly available data from the World Health Organization, COVID-19 can induce or exacerbate systemic inflammation. Currently, there is a lack of effective clinical markers to diagnose asymptomatic COVID-19-related systemic inflammation. The present invention provides that ADGRE3 mRNA or protein can be used to diagnose such cases.

In some embodiments, the biomarker is used to prepare a reagent for diagnosing whether an individual has systemic inflammation due to respiratory syncytial virus (RSV) infection.

Further, such diagnosis includes determining whether the individual has acute RSV infection-induced systemic inflammation or has recovered from RSV-induced systemic inflammation.

There is a statistically significant difference in neutrophil ADGRE3 mRNA expression levels between acute RSV infection cases and recovery-stage patients. Therefore, ADGRE3 mRNA detection can monitor RSV-related disease progression and provide clinical guidance.

In some embodiments, the biomarker is used to prepare a reagent for diagnosing whether an individual has systemic inflammation due to cytomegalovirus (CMV) infection.

In some embodiments, the biomarker is used to prepare a reagent for diagnosing whether an individual has systemic inflammation due to Epstein-Barr virus (EBV) infection.

In some embodiments, the biomarker is used to prepare a reagent for differential diagnosis among healthy individuals, patients with non-infectious systemic inflammatory response syndrome (SIRS), and patients with sepsis. The biomarker comprises ADGRE3 mRNA or fragments thereof; or ADGRE3 protein or fragments thereof.

There are statistically significant differences in leukocyte ADGRE3 mRNA transcription levels among healthy individuals, patients with non-infectious systemic inflammatory response syndrome (SIRS), and patients with sepsis, showing a descending trend in that order. The diagnostic performance has been clinically validated, with the area under the curve (AUC) for distinguishing healthy individuals from sepsis patients reaching 0.920, the AUC for distinguishing sepsis from non-infectious SIRS reaching 0.864, and the AUC for diagnosing sepsis from all sample types reaching 0.862. These results demonstrate that ADGRE3 mRNA can accurately distinguish non-infectious SIRS from sepsis. Similar results are obtained with ADGRE3 protein or fragments thereof.

In some embodiments, the biomarker is used to prepare a reagent for diagnosing whether an individual has bacterial systemic inflammation, wherein the biomarker comprises ADGRE3 mRNA or fragments thereof.

Third aspect of the invention: The present invention provides a method for diagnosing whether an individual has systemic inflammation, the method comprising: providing a sample to be tested from the individual; detecting the level of ADGRE3 mRNA or fragments thereof; or ADGRE3 protein or fragments thereof in the sample; comparing the detected level with a predetermined threshold; determining the individual does not have systemic inflammation if the level is greater than or equal to the threshold, or determining the individual has systemic inflammation if the level is below the threshold.

In some embodiments, detecting ADGRE3 mRNA or a fragment thereof in a sample refers to measuring the level of ADGRE3 mRNA or a fragment thereof in leukocytes in the individual's sample; In some embodiments, detecting ADGRE3 protein or a fragment thereof in a sample refers to measuring the level of ADGRE3 protein or a fragment thereof in neutrophils and/or monocytes in the individual's sample.

In some embodiments, the sample includes one or more of peripheral blood, urine, secretions, pus, body fluids, cerebrospinal fluid, or fresh tissue.

In some embodiments, the systemic inflammation comprises infectious systemic inflammation or non-infectious systemic inflammation.

In some embodiments, the infectious systemic inflammation comprises systemic inflammation caused by any one or more of bacterial infection, viral infection, fungal infection, or parasitic infection.

In some embodiments, the viral infection comprises any one or more of influenza virus infection, dengue virus infection, COVID-19 infection, respiratory syncytial virus infection, cytomegalovirus infection, Epstein-Barr virus infection, or SFTSV infection.

In some embodiments, the non-infectious systemic inflammation comprises systemic inflammation caused by any one or more of autoimmune diseases, transplant rejection, inflammatory bowel disease, allergic reactions, metabolic diseases, or tissue injury-induced inflammation.

In some embodiments, the autoimmune disease comprises any one or more of systemic lupus erythematosus, rheumatoid arthritis, vasculitis, or ankylosing spondylitis.

In some embodiments, the transplant rejection-induced systemic inflammation comprises systemic inflammation caused by any one or more of bone marrow transplantation, kidney transplantation, or liver transplantation.

In some embodiments, the systemic inflammation comprises sepsis.

In some embodiments, the present invention provides a method for diagnosing whether a subject is suffering from systemic inflammation caused by bacterial infection or viral infection, the method comprising: providing a test sample; detecting the level of ADGRE3 mRNA or a fragment thereof, or ADGRE3 protein or a fragment thereof in the sample; comparing the detected level with a predetermined threshold, wherein if the detected level is greater than or equal to the threshold, the subject is diagnosed as not having systemic inflammation caused by bacterial or viral infection; and if the detected level is less than the threshold, the subject is diagnosed as having systemic inflammation caused by bacterial or viral infection.

In some embodiments, the present invention provides a method for diagnosing whether a subject is suffering from systemic inflammation caused by Gram-positive bacterial infection or H1N1 infection, the method comprising: providing a test sample; detecting the level of ADGRE3 mRNA or a fragment thereof, or ADGRE3 protein or a fragment thereof, in the sample; comparing the detected level with a predetermined threshold, wherein if the detected level is greater than or equal to the threshold, the subject is diagnosed as not having systemic inflammation caused by Gram-positive bacterial infection or H1N1 infection; and if the detected level is less than the threshold, the subject is diagnosed as having systemic inflammation caused by Gram-positive bacterial infection or H1N1 infection.

On the other hand, the present invention provides a method for differential diagnosis of whether a patient with systemic inflammation is suffering from Gram-positive bacterial infection or H1N1 infection, the method comprising: providing a test sample; detecting the level of ADGRE3 mRNA or a fragment thereof, or ADGRE3 protein or a fragment thereof, in the sample; comparing the detected level with a predetermined threshold, wherein if the detected level is greater than the threshold, the patient is diagnosed as having systemic inflammation with a Gram-positive bacterial infection, and if the detected level is less than the threshold, the patient is diagnosed as having an H1N1 infection.

On the other hand, the present invention provides a method for diagnosing whether a subject is suffering from dengue virus-associated systemic inflammation, the method comprising: providing a test sample; detecting the level of ADGRE3 mRNA or a fragment thereof, or ADGRE3 protein or a fragment thereof, in the sample; comparing the detected level with a predetermined threshold, wherein if the detected level is greater than or equal to the threshold, the subject is diagnosed as not having dengue virus-associated systemic inflammation, and if the detected level is less than the threshold, the subject is diagnosed as having dengue virus-associated systemic inflammation.

On the other hand, the present invention provides a method for diagnosing whether a subject is suffering from systemic inflammation caused by COVID-19 infection, the method comprising: providing a test sample; detecting the level of ADGRE3 mRNA or a fragment thereof, or ADGRE3 protein or a fragment thereof, in the sample; comparing the detected level with a predetermined threshold, wherein if the detected level is greater than or equal to the threshold, the subject is diagnosed as not having COVID-19-associated systemic inflammation, and if the detected level is less than the threshold, the subject is diagnosed as having COVID-19-associated systemic inflammation.

Furthermore, the diagnosis of whether the subject is suffering from COVID-19-associated systemic inflammation includes diagnosing a subject with asymptomatic COVID-19-associated systemic inflammation or symptomatic COVID-19-associated systemic inflammation.

On the other hand, the present invention provides a method for diagnosing whether a subject is suffering from systemic inflammation caused by respiratory syncytial virus (RSV) infection, the method comprising: providing a test sample; detecting the level of ADGRE3 mRNA or a fragment thereof, or ADGRE3 protein or a fragment thereof, in the sample; comparing the detected level with a predetermined threshold, wherein if the detected level is greater than or equal to the threshold, the subject is diagnosed as not having RSV-associated systemic inflammation, and if the detected level is less than the threshold, the subject is diagnosed as having RSV-associated systemic inflammation.

Furthermore, the diagnosis of whether the subject is suffering from RSV-associated systemic inflammation includes diagnosing whether the subject is undergoing acute RSV-associated systemic inflammation or recovering from RSV-associated systemic inflammation.

On the other hand, the present invention provides a method for diagnosing whether a subject is suffering from systemic inflammation caused by cytomegalovirus (CMV) infection, the method comprising: providing a test sample; detecting the level of ADGRE3 mRNA or a fragment thereof, or ADGRE3 protein or a fragment thereof, in the sample; comparing the detected level with a predetermined threshold, wherein if the detected level is greater than or equal to the threshold, the subject is diagnosed as not having CMV-associated systemic inflammation, and if the detected level is less than the threshold, the subject is diagnosed as having CMV-associated systemic inflammation.

On the other hand, the present invention provides a method for diagnosing whether a subject is suffering from systemic inflammation caused by Epstein-Barr virus (EBV) infection, the method comprising: providing a test sample; detecting the level of ADGRE3 mRNA or a fragment thereof, or ADGRE3 protein or a fragment thereof, in the sample; comparing the detected level with a predetermined threshold, wherein if the detected level is greater than or equal to the threshold, the subject is diagnosed as not having EBV-associated systemic inflammation, and if the detected level is less than the threshold, the subject is diagnosed as having EBV-associated systemic inflammation.

Additionally, the present invention provides a method for distinguishing whether an individual is a healthy subject, a patient with non-infectious systemic inflammatory response syndrome (SIRS), or a patient with sepsis, the method comprising: providing a test sample; detecting the level of ADGRE3 mRNA or a fragment thereof, or ADGRE3 protein or a fragment thereof, in the sample; and comparing the detected level with predetermined thresholds, the thresholds including a threshold for distinguishing healthy subjects from non-infectious SIRS patients, and a threshold for distinguishing non-infectious SIRS patients from sepsis patients.

In some embodiments, when the detected level is greater than or equal to the threshold distinguishing healthy subjects from non-infectious SIRS patients, the individual is diagnosed as a healthy subject.

In some embodiments, when the detected level is less than the threshold distinguishing non-infectious SIRS patients from sepsis patients, the individual is diagnosed as a sepsis patient.

In some embodiments, when the detected level is lower than the threshold distinguishing healthy subjects from non-infectious SIRS patients and higher than the threshold distinguishing non-infectious SIRS patients from sepsis patients, the individual is diagnosed as a non-infectious SIRS patient.

On the other hand, the present invention provides a method for diagnosing whether an individual is suffering from systemic inflammation caused by transplant organ rejection, the method comprising: providing a test sample; detecting the level of ADGRE3 mRNA or a fragment thereof, or ADGRE3 protein or a fragment thereof, in the sample; and comparing the detected level with a predetermined threshold, wherein if the detected level is greater than or equal to the threshold, the individual is diagnosed as not having transplant rejection-induced systemic inflammation, and if the detected level is less than the threshold, the individual is diagnosed as having transplant rejection-induced systemic inflammation.

In the above studies, we discovered and validated that ADGRE3 mRNA, or ADGRE3 protein or fragments thereof, can be used to diagnose systemic inflammation, as well as to diagnose other related diseases. ADGRE3 mRNA is transcribed from the ADGRE3 gene, and ADGRE3 protein is translated from said mRNA; however, the protein level is not solely determined by the mRNA level. The level of ADGRE3 protein is also affected by factors such as protein-protein interactions and degradation. Despite this, our functional validation also demonstrated that ADGRE3 protein or fragments thereof have diagnostic utility for systemic inflammation.

On the other hand, the present invention provides the use of a biomarker in the preparation of a reagent for diagnosing systemic inflammation, wherein the biomarker comprises ADGRE3 protein or a fragment thereof, or an amino acid sequence or peptide fragment corresponding to ADGRE3.

The diagnosis of systemic inflammation includes one or more of the following: diagnosing whether an individual is suffering from systemic inflammation, predicting the risk of developing systemic inflammation, monitoring disease progression in patients with systemic inflammation, assessing prognosis in patients with systemic inflammation, or providing medication guidance for patients with systemic inflammation.

The reagent for diagnosing systemic inflammation is used to detect ADGRE3 protein or a fragment thereof in a biological sample from a subject. Preferably, the ADGRE3 protein or fragment is present on or in neutrophils or monocytes within the sample.

ADGRE3 protein is primarily expressed in neutrophils and is also expressed in monocytes. However, the detection of ADGRE3 protein or fragments in neutrophils or monocytes does not limit the nature of the test sample. Any sample containing neutrophils or monocytes, or substances originating from within neutrophils or monocytes (referring to materials derived from neutrophils or monocytes), may be used as a test sample for diagnosing systemic inflammation based on neutrophil- or monocyte-derived ADGRE3 protein or fragments.

Detecting ADGRE3 protein or fragments thereof in neutrophils or monocytes refers to detecting ADGRE3 protein or fragments derived from neutrophils or monocytes. The ADGRE3 protein or fragments may be located on the cell membrane, outside the cell, or be transmembrane. The detection of such levels may be carried out by flow cytometry or other known techniques.

In some embodiments, the sample includes one or more of peripheral blood, urine, secretions, pus, bodily fluids, cerebrospinal fluid, or fresh tissue.

By detecting the level or amount of ADGRE3 protein in neutrophils from healthy individuals and clinical patients, significant differences are observed between the neutrophil ADGRE3 protein expression levels of various types of systemic inflammation patients and healthy individuals. Therefore, systemic inflammation can be diagnosed by measuring the amount of neutrophil ADGRE3 protein. The expression levels of ADGRE3 protein in neutrophils and monocytes are correlated, so systemic inflammation can also be diagnosed by measuring ADGRE3 protein expression levels in monocytes.

In combination with multiple known inflammatory markers, the present invention finds that ADGRE3 protein exhibits higher diagnostic sensitivity. When interference cases from patients with localized inflammation are included, diagnostic validation among healthy individuals, localized inflammation patients, and systemic inflammation patients shows that ADGRE3 protein can correctly classify these two types of inflammation, demonstrating high specificity of ADGRE3 protein for diagnosing systemic inflammation.

In some embodiments, the systemic inflammation includes infectious systemic inflammation or non-infectious systemic inflammation.

In some embodiments, the infectious systemic inflammation includes one or more of bacterial infection, viral infection, fungal infection, or parasitic infection.

In some embodiments, the viral infection includes one or more of influenza virus infection, coronavirus infection (COVID-19), dengue virus infection, severe fever with thrombocytopenia syndrome virus (SFTSV) infection, respiratory syncytial virus infection, cytomegalovirus infection, or Epstein-Barr virus infection.

In some embodiments, the non-infectious systemic inflammation includes one or more of autoimmune diseases, transplant organ rejection, inflammatory bowel disease, allergic reactions, metabolic diseases, or tissue injury-induced inflammation.

In some embodiments, the autoimmune diseases include one or more of systemic lupus erythematosus, rheumatoid arthritis, vasculitis, ankylosing spondylitis, or autoimmune hepatitis.

Besides systemic inflammation caused by systemic lupus erythematosus, rheumatoid arthritis, vasculitis, ankylosing spondylitis, and autoimmune hepatitis, systemic inflammation caused by other autoimmune diseases can also be diagnosed by detecting ADGRE3 mRNA or ADGRE3 mRNA fragments, ADGRE3 protein or ADGRE3 protein fragments as discovered in the present invention.

In some embodiments, the transplant organ rejection-induced inflammation includes systemic inflammation caused by rejection reactions after liver transplantation, kidney transplantation, or bone marrow transplantation.

In some embodiments, the systemic inflammation includes sepsis.

In some embodiments, the sepsis is caused by one or more infections selected from pulmonary infection, biliary tract infection, urinary tract infection, digestive tract infection, bloodstream infection, reproductive tract infection, abdominal infection, central nervous system infection, skin and soft tissue infection, or suppurative osteomyelitis.

Besides sepsis caused by the aforementioned foci, sepsis caused by any other lesion can also be diagnosed by detecting ADGRE3 mRNA or ADGRE3 mRNA fragments, ADGRE3 protein or ADGRE3 protein fragments as discovered herein.

In some embodiments, the sepsis includes sepsis caused by Gram-positive bacterial infection, Gram-negative bacterial infection, or fungal infection, one or more of these.

In some embodiments, the sepsis includes septic shock.

In some embodiments, the present invention provides the use of a biomarker for the preparation of a reagent for differentiating and diagnosing an individual as a healthy subject or a viral infection-induced systemic inflammation patient, wherein the biomarker comprises ADGRE3 protein or a fragment thereof, or an amino acid sequence or peptide fragment corresponding to ADGRE3.

In some embodiments, the reagent is further capable of differentiating and diagnosing viral infection-induced systemic inflammation patients as mild, severe, or critical cases.

In some embodiments, the present invention provides the use of a biomarker for preparing a reagent for diagnosing whether an individual has systemic lupus erythematosus-associated systemic inflammation, wherein the biomarker comprises ADGRE3 protein or a fragment thereof, or an amino acid sequence or peptide fragment corresponding to ADGRE3.

In some embodiments, the present invention provides the use of a biomarker for preparing a reagent for diagnosing systemic lupus erythematosus patients as being in a stable or active disease phase, wherein the biomarker comprises ADGRE3 protein or a fragment thereof, or an amino acid sequence or peptide fragment corresponding to ADGRE3.

In some embodiments, the present invention provides the use of a biomarker for preparing a reagent for diagnosing whether an individual has rheumatoid arthritis-associated systemic inflammation, wherein the biomarker comprises ADGRE3 protein or a fragment thereof, or an amino acid sequence or peptide fragment corresponding to ADGRE3.

In some embodiments, the present invention provides the use of a biomarker for preparing a reagent for diagnosing rheumatoid arthritis systemic inflammation patients as either stable phase patients or active phase patients. The biomarker comprises ADGRE3 protein or a fragment thereof, or an amino acid sequence or peptide fragment corresponding to ADGRE3.

Systemic lupus erythematosus and rheumatoid arthritis can be divided into stable phase and active phase according to disease progression. The stable phase refers to a period during which the condition no longer shows significant improvement or worsening, while the active phase refers to any period other than the stable phase. Treatment regimens differ between stable and active phases; for example, stable phase systemic lupus erythematosus patients require reduced medication dosage while maintaining relatively frequent monitoring of the condition. Clinically, it is necessary to diagnose the stable and active phases of systemic lupus erythematosus and rheumatoid arthritis. However, currently there are no indicators capable of providing clear diagnosis. The present invention finds that ADGRE3 protein can serve as a biomarker to diagnose the stable and active phases of systemic lupus erythematosus and rheumatoid arthritis.

In some embodiments, the present invention provides the use of a biomarker for preparing a reagent for differentiating and diagnosing an individual as a healthy subject, a sepsis patient, or a septic shock patient. The biomarker includes ADGRE3 protein or a fragment thereof, or an amino acid sequence or peptide fragment corresponding to ADGRE3.

Sepsis can be categorized by severity into sepsis, severe sepsis, and septic shock. Septic shock patients are critically ill and require substantial medical resources for urgent treatment. Therefore, when sepsis occurs, it is necessary to classify patients according to severity. The present invention finds that ADGRE3 protein can differentiate and diagnose septic shock, assisting clinical patient classification.

In some embodiments, the present invention provides the use of a biomarker for preparing a reagent for predicting the mortality risk of sepsis patients. The biomarker includes ADGRE3 protein or a fragment thereof, or an amino acid sequence or peptide fragment corresponding to ADGRE3.

The invention further finds that mortality risk in sepsis patients is highly correlated with ADGRE3 protein levels, and the disease progression of sepsis also shows high correlation with changes in ADGRE3 protein amount.

In some embodiments, the use further includes: if the expression level of neutrophil ADGRE3 protein on day 5 after diagnosis is higher than that on day 3, the individual's sepsis is predicted to improve; otherwise, sepsis is predicted not to improve or to result in death.

On the other hand, the invention provides a biomarker panel for preparing a reagent to diagnose whether a systemic inflammation patient is suffering from bacteria-induced systemic inflammation. The biomarker panel includes ADGRE3 protein or a fragment thereof, or an amino acid sequence or peptide fragment corresponding to ADGRE3, and further includes one or more traditional biomarkers specific for diagnosing bacterial inflammation.

The traditional biomarkers specific for diagnosing bacterial inflammation are those whose levels change significantly when bacterial inflammation occurs, but do not change significantly when there is no inflammation or when inflammation is non-bacterial, thus providing specificity for bacterial inflammation. Several such biomarkers are known, including CD64, PCT, HBP, and others.

In some embodiments, the bacterial inflammation-specific biomarkers include one or more of CD64, PCT, and HBP.

In some embodiments, when the amount of ADGRE3 protein or fragment thereof, or the corresponding amino acid sequence or peptide fragment in the sample is below a preset threshold, and the bacterial inflammation-specific biomarker level in the sample is outside the range typical for healthy individuals, the patient is diagnosed as having bacteria-induced systemic inflammation.

In some embodiments, when the amount of ADGRE3 protein or fragment thereof, or the corresponding amino acid sequence or peptide fragment in the sample is below the preset threshold, but the bacterial inflammation-specific biomarker level in the sample is within the range typical for healthy individuals, the patient is diagnosed as having systemic inflammation caused by viral infection or non-infectious disease.

In some embodiments, the preset threshold corresponds to the level of ADGRE3 protein or fragment thereof, or the corresponding amino acid sequence or peptide fragment in healthy individuals.

The invention measures both neutrophil ADGRE3 protein levels and bacterial inflammation-specific biomarker levels in the sample and finds that when ADGRE3 protein levels decrease and bacterial inflammation-specific biomarker levels significantly change or increase, the patient is classified as having bacterial systemic inflammation. If bacterial inflammation-specific biomarker levels remain normal, the patient is classified as having non-bacterial systemic inflammation. Thus, the amount of ADGRE3 protein or fragment thereof, combined with bacterial inflammation-specific biomarkers, can distinguish between systemic inflammation caused by bacterial infection and that caused by viral infection or non-infectious disease.

Regarding the levels of biomarkers specific for diagnosing bacterial inflammation, levels outside the normal range for healthy individuals can include either elevated levels (above the healthy range) or decreased levels (below the healthy range). Either of these changes in the bacterial inflammation-specific biomarkers indicates the occurrence of bacterial inflammation. For example, an elevated nCD64 measurement indicates bacterial inflammation.

On the other hand, the present invention provides a method for diagnosing systemic inflammation, comprising: providing a test sample; detecting the content of ADGRE3 protein or an ADGRE3 protein fragment, or the corresponding amino acid sequence or peptide fragment in the sample; comparing the detected content to a predetermined threshold; diagnosing the individual as not having systemic inflammation if the detected content is greater than or equal to the threshold, or diagnosing the individual as having systemic inflammation if the detected content is less than the threshold.

Additionally, the present invention provides a method for diagnosing whether an individual has systemic inflammation, comprising: providing a test sample; detecting the content of ADGRE3 protein or an ADGRE3 protein fragment, or the corresponding amino acid sequence or peptide fragment in the sample; comparing the detected content to a predetermined threshold; diagnosing the individual as not having systemic inflammation if the detected content is greater than or equal to the threshold, or diagnosing the individual as having systemic inflammation if the detected content is less than the threshold.

Regarding the detection of ADGRE3 protein or protein fragment levels in the sample, the invention employs flow cytometry gating on cells in the sample, allowing targeted detection of ADGRE3 protein or its fragments specifically on neutrophils or monocytes in the sample. The ADGRE3 protein or fragment may be extracellular, transmembrane, or intracellular; all these forms can be quantified by flow cytometry. The use of any known method combined with flow cytometry to detect ADGRE3 protein or fragment levels in the sample to diagnose systemic inflammation falls within the scope of this invention.

Concerning the threshold setting for ADGRE3 protein or fragment levels, due to variability in experiments, different flow cytometers, and detection methods, direct measurements such as mean fluorescence intensity (MFI) vary. Therefore, the invention adopts the neutrophil ADGRE3 expression index (nADGRE3), defined as: nADGRE3 = (MFI of ADGRE3 protein on patient neutrophils) / (MFI of ADGRE3 protein on healthy control neutrophils).

The invention found that an nADGRE3 threshold value of 0.8361 achieves the highest sensitivity, specificity, and accuracy in diagnosing systemic inflammation. The use of any other known threshold-setting methods combined with ADGRE3 protein or fragment detection for diagnosing systemic inflammation is included within the scope of the invention.

Preferably, ADGRE3 protein or its fragments are present on or within neutrophils or monocytes in the sample.

In some embodiments, the sample includes one or more of peripheral blood, urine, secretions, pus, bodily fluids, cerebrospinal fluid, or fresh tissue.

In some embodiments, systemic inflammation includes infectious or non-infectious systemic inflammation.

In some embodiments, infectious systemic inflammation includes systemic inflammation caused by any one or more of bacterial infection, viral infection, fungal infection, or parasitic infection.

In some embodiments, viral infections include any one or more of influenza virus infection, novel coronavirus infection, dengue virus infection, or SFTSV infection.

In some embodiments, non-infectious systemic inflammation includes systemic inflammation caused by any one or more of autoimmune diseases, organ transplant rejection, inflammatory bowel disease, allergic reactions, metabolic diseases, or inflammation caused by tissue injury.

In some embodiments, autoimmune diseases include any one or more of systemic lupus erythematosus, rheumatoid arthritis, vasculitis, ankylosing spondylitis, or autoimmune hepatitis.

In some embodiments, organ transplant rejection-induced systemic inflammation includes systemic inflammation caused by any one or more of bone marrow transplantation, kidney transplantation, or liver transplantation.

In some embodiments, systemic inflammation includes sepsis.

In some embodiments, sepsis includes any one or more of Gram-positive bacterial sepsis, Gram-negative bacterial sepsis, or fungal sepsis.

In some embodiments, sepsis includes septic shock.

In some embodiments, the present invention provides a method for diagnosing whether an individual has viral infection-induced systemic inflammation, the method comprising: providing a test sample; detecting the content of ADGRE3 protein or an ADGRE3 protein fragment, or the corresponding amino acid sequence or peptide fragment in the sample; comparing the detected content to a predetermined threshold; diagnosing the individual as not having viral infection-induced systemic inflammation if the content is greater than or equal to the threshold, or diagnosing the individual as having viral infection-induced systemic inflammation if the content is less than the threshold.

In some embodiments, the invention provides a method to classify viral infection systemic inflammation patients as mild, severe, or critical cases, the method comprising: providing a test sample; detecting the content of ADGRE3 protein or an ADGRE3 protein fragment, or the corresponding amino acid sequence or peptide fragment; comparing the detected content to predetermined thresholds that differentiate mild from severe and severe from critical cases.

In some embodiments, when the detected content is greater than the threshold that distinguishes mild from severe, the patient is classified as mild.

In some embodiments, when the detected content is less than the threshold that distinguishes severe from critical, the patient is classified as critical.

In some embodiments, when the detected content is less than the mild/severe threshold but greater than the severe/critical threshold, the patient is classified as severe.

On the other hand, the invention provides a method to classify systemic lupus erythematosus systemic inflammation patients into stable or active phase, the method comprising: providing a test sample; detecting the content of ADGRE3 protein or protein fragment or corresponding amino acid sequence or peptide fragment; comparing the detected content to a predetermined threshold; classifying the patient as stable phase if the content is greater than or equal to the threshold, or as active phase if the content is less than the threshold.

On the other hand, the present invention provides a method for classifying whether a rheumatoid arthritis-associated systemic inflammation patient is in a stable phase or an active phase, the method comprising: providing a test sample; detecting ADGRE3 protein or a fragment thereof in the sample; comparing the detected level with a predetermined threshold; and classifying the patient as being in the stable phase when the detected level is greater than or equal to the threshold, or as being in the active phase when the detected level is less than the threshold.

On the other hand, the invention provides a method for diagnosing whether an individual has sepsis, comprising: providing a test sample; detecting ADGRE3 protein or protein fragment content, or corresponding amino acid sequence or peptide fragment content; comparing the detected content to a predetermined threshold; diagnosing the individual as not having sepsis if the content is greater than or equal to the threshold, or as having sepsis if the content is less than the threshold.

On the other hand, the present invention provides a method for predicting the mortality risk of a sepsis patient, the method comprising: providing a test sample; detecting the content of ADGRE3 protein or an ADGRE3 protein fragment in the sample, or the content of the corresponding amino acid sequence or peptide fragment of ADGRE3; comparing the detected content to a predetermined threshold, wherein when the detected content is greater than or equal to the threshold, the sepsis patient is predicted to have a low mortality risk, and when the detected content is less than the threshold, the sepsis patient is predicted to have a high mortality risk.

Further, the method comprises: if the expression level of neutrophil ADGRE3 protein on day 5 after diagnosis is higher than that on day 3, predicting that the sepsis of the patient is improved; otherwise, predicting that the sepsis of the patient is unimproved or that the patient dies.

On the other hand, the present invention provides a method for diagnosing whether an individual has systemic inflammation caused by organ transplant rejection, the method comprising: providing a test sample; detecting the content of ADGRE3 protein or an ADGRE3 protein fragment in the sample, or the content of the corresponding amino acid sequence or peptide fragment of ADGRE3; comparing the detected content to a predetermined threshold, wherein when the detected content is greater than or equal to the threshold, diagnosing that the individual does not have systemic inflammation caused by organ transplant rejection, and when less than the threshold, diagnosing that the individual has systemic inflammation caused by organ transplant rejection.

On the other hand, the present invention provides a method for diagnosing whether an individual has bacterial systemic inflammation, the method comprising: providing a test sample; detecting the content of ADGRE3 protein or an ADGRE3 protein fragment in the sample, or the content of the corresponding amino acid sequence or peptide fragment of ADGRE3; comparing the detected content to a predetermined threshold, wherein when the detected content is greater than or equal to the threshold, diagnosing that the individual does not have bacterial systemic inflammation;
when less than the threshold, detecting one or more biomarkers specifically diagnosing bacterial inflammation in the sample; if the biomarker content is outside the range found in healthy individuals, diagnosing that the individual has bacterial systemic inflammation; if the biomarker content is within the healthy range, diagnosing that the individual does not have bacterial systemic inflammation.

In some embodiments, the biomarkers specifically diagnosing bacterial inflammation include one or more of CD64, PCT, and HBP.

The ADGRE3 gene corresponds to multiple mRNA transcripts and transcript variants; any ADGRE3 mRNA or its fragments can serve as a biomarker of the present invention; multiple ADGRE3 proteins translated from these mRNAs may have different amino acid sequences, and any ADGRE3 protein or its fragments translated from any ADGRE3 mRNA can serve as a biomarker of the present invention.

In some embodiments, the ADGRE3 mRNAs comprise any one or more of the nucleotide sequences as shown in Ref Seq database entries XM_054322398.1, XM_047439546.1, XM_011528374.3, NM_001289158.2, NM_001289159.2, and NM_032571.5.

In some embodiments, the ADGRE3 protein comprises the amino acid sequence shown as SEQ ID NO:1; or the ADGRE3 protein is translated from any one of the mRNAs XM_054322398.1, XM_047439546.1, XM_011528374.3, NM_001289158.2, NM_001289159.2, or NM_032571.5.

### BENEFICIAL EFFECTS OF THE INVENTION

For the first time, ADGRE3 was identified as a single biomarker useful for diagnosing systemic inflammation. The transcription level or expression level of ADGRE3 mRNA or ADGRE3 protein in white blood cells, neutrophils, or monocytes can distinguish ordinary inflammation from systemic inflammation with high specificity. It has a high sensitivity for indicating systemic inflammation, supporting its utility as a biomarker, and its specificity for diagnosing systemic inflammation has been clinically validated.

### BRIEF DESCRIPTION OF THE DRAWINGS

Figure 1: Volcano plot of differentially expressed genes in peripheral blood between sepsis patients and non-infectious systemic inflammatory response patients.
Figure 2: Volcano plot of differentially expressed genes in peripheral blood between sepsis patients and healthy individuals.
Figure 3: Comparison of leukocyte ADGRE3 mRNA transcription levels among healthy controls, bacterial infection systemic inflammation patients, and viral infection systemic inflammation patients.
Figure 4: Comparison of leukocyte ADGRE3 mRNA transcription levels among healthy controls, Gram-negative bacterial infection systemic inflammation patients, and H1N1 infection systemic inflammation patients.
Figure 5: Comparison of leukocyte ADGRE3 mRNA transcription levels between healthy controls and dengue systemic inflammation patients.
Figure 6: Comparison of leukocyte ADGRE3 mRNA transcription levels among healthy controls, asymptomatic COVID-19 infection systemic inflammation patients, and symptomatic COVID-19 infection-induced systemic inflammation patients.
Figure 7: Comparison of leukocyte ADGRE3 mRNA transcription levels among healthy controls, RSV acute infection systemic inflammation patients, and individuals recovered from RSV infection systemic inflammation.
Figure 8: Comparison of leukocyte ADGRE3 mRNA transcription levels between healthy controls and cytomegalovirus infection systemic inflammation patients.
Figure 9: Comparison of leukocyte ADGRE3 mRNA transcription levels between healthy controls and Epstein-Barr virus infection systemic inflammation patients.
Figure 10: Comparison of leukocyte ADGRE3 mRNA transcription levels between healthy controls and systemic inflammation patients caused by liver transplant rejection.
Figure 11: Comparison of leukocyte ADGRE3 mRNA transcription levels among healthy controls, non-infectious systemic inflammatory response syndrome patients, and sepsis patients.
Figure 12: ROC curve of leukocyte ADGRE3 mRNA transcription levels among healthy controls and sepsis patients.
Figure 13: ROC curve of leukocyte ADGRE3 mRNA transcription levels for differential diagnosis between non-infectious systemic inflammatory response syndrome and sepsis patients.
Figure 14: Comparison of leukocyte ADGRE3 mRNA transcription levels among healthy controls, influenza A and B virus infection systemic inflammation patients, and novel coronavirus infection systemic inflammation patients.
Figure 15: Comparison of leukocyte ADGRE3 mRNA transcription levels (ADGRE3 protein) among healthy controls, systemic lupus erythematosus associated systemic inflammation patients, rheumatoid arthritis associated systemic inflammation patients, vasculitis associated systemic inflammation patients, and ankylosing spondylitis associated systemic inflammation patients.
Figure 16: Comparison of leukocyte ADGRE3 mRNA transcription levels between healthy controls and sepsis patients.
Figure 17: Comparison of neutrophil ADGRE3 protein expression levels in peripheral blood among healthy controls, influenza A and B virus infection systemic inflammation patients, COVID-19 infection systemic inflammation patients, dengue systemic inflammation patients, and severe fever with thrombocytopenia syndrome virus (SFTSV) infection systemic inflammation patients.
Figure 18: ROC curve of neutrophil ADGRE3 protein expression for differential diagnosis between healthy individuals and viral infection systemic inflammation patients.
Figure 19: Comparison of neutrophil ADGRE3 protein expression levels among healthy controls, mild, severe, and critical viral infection systemic inflammation patients.
Figure 20: Comparison of neutrophil ADGRE3 protein expression levels among autoimmune disease systemic inflammation patients (systemic lupus erythematosus, rheumatoid arthritis, vasculitis, ankylosing spondylitis, autoimmune hepatitis).
Figure 21: Comparison of neutrophil ADGRE3 protein expression levels among healthy controls, systemic inflammation patients caused by kidney transplantation, and systemic inflammation patients caused by bone marrow transplantation.
Figure 22: ROC curve of neutrophil ADGRE3 protein expression levels for differential diagnosis between healthy individuals and non-infectious systemic inflammation.
Figure 23: Comparison of neutrophil ADGRE3 protein expression levels between healthy controls and sepsis patients.
Figure 24: ROC curve of neutrophil ADGRE3 protein expression for differential diagnosis between healthy individuals and sepsis patients.
Figure 25: Comparison of neutrophil ADGRE3 protein expression levels among healthy controls, Gram-positive bacterial sepsis, Gram-negative bacterial sepsis, and fungal sepsis patients.
Figure 26: Comparison of neutrophil ADGRE3 protein expression levels among healthy controls, sepsis patients, and septic shock patients.
Figure 27: Comparison of neutrophil ADGRE3 protein expression between sepsis survivors and sepsis deceased patients.
Figure 28: ROC curve of neutrophil ADGRE3 protein for predicting mortality risk in sepsis patients.
Figure 29: Comparison of neutrophil ADGRE3 protein expression between sepsis patients with clinical improvement and those without clinical improvement or death.
Figure 30: Comparison of neutrophil ADGRE3 protein expression among healthy controls and local inflammation patients (hepatitis B, hepatitis C, hepatitis E patients).
Figure 31: Flowchart of classification of bacterial infection-induced systemic inflammation patients based on neutrophil ADGRE3 and other inflammatory markers.
Figure 32: Flowchart of classification of viral infection-induced systemic inflammation patients based on neutrophil ADGRE3 and other inflammatory markers.
Figure 33: Flowchart of classification of autoimmune disease and transplant rejection-induced systemic inflammation patients based on neutrophil ADGRE3 and other inflammatory markers.
Figure 34: Diagnostic classification flowchart of suspected systemic inflammatory response patients based on neutrophil ADGRE3 and other bacterial inflammation markers.

### Explanation of the marks and contents in the figures

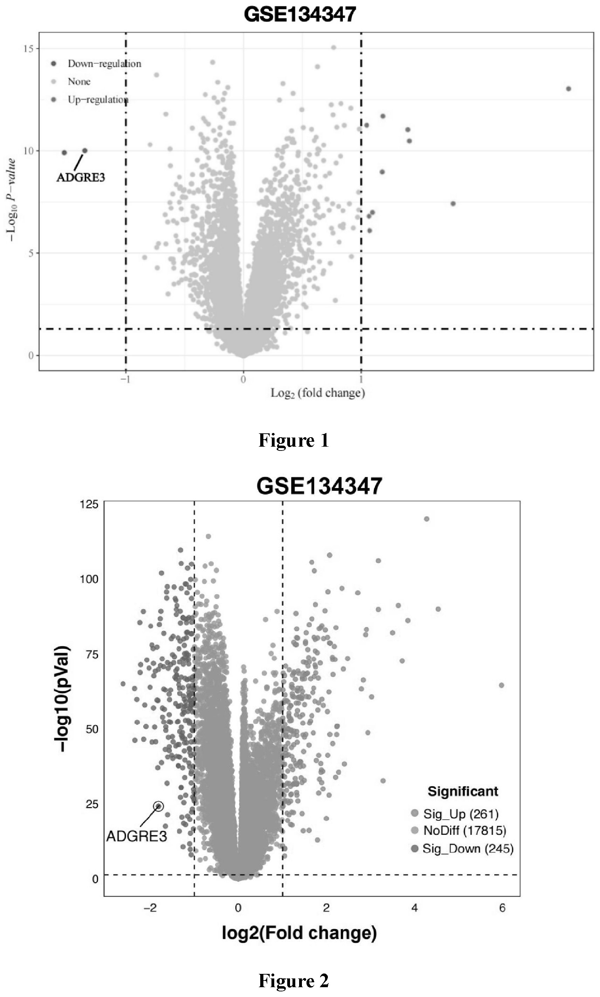

In Figures 1 and 2, each point corresponds to a gene. A point located to the left of the origin on the horizontal axis indicates a downregulation of the corresponding gene expression, while a point to the right of the origin indicates upregulation of the gene expression. The color of the points represents changes in gene expression greater than 2-fold with statistical significance (p < 0.05): blue indicates expression decreased by more than 2-fold, and red indicates expression significantly increased by more than 2-fold. The farther the point is from the origin on the horizontal axis, the greater the change in gene expression. An absolute horizontal coordinate value greater than or equal to 1 represents a change greater than 2-fold, and an absolute value greater than or equal to 2 represents a change greater than 4-fold.

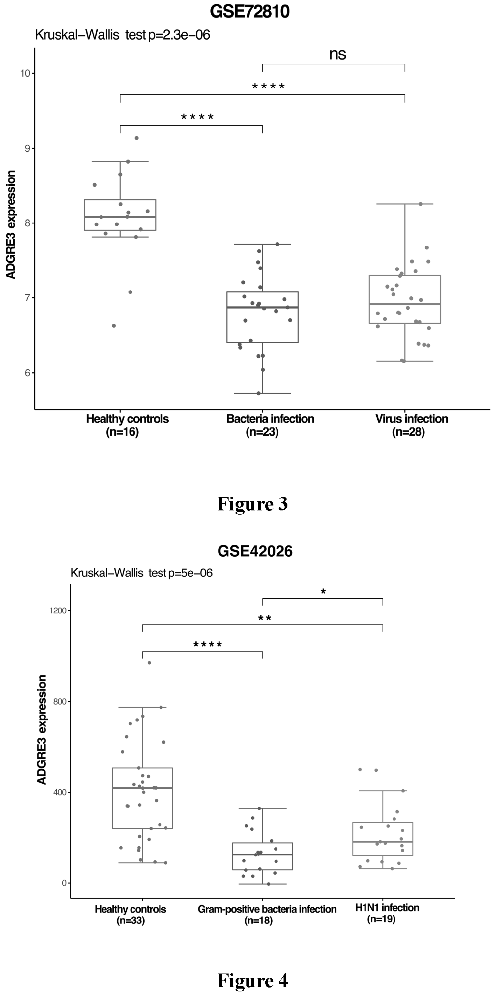

As shown in Figure 3, in the graphs related to mRNA expression levels, the vertical axis represents mRNA expression levels, specifically the copy number of mRNA. "GSE+number" refers to the dataset identification number in the GEO database.

In graphs related to the level of ADGRE3 protein encoded by mRNA, the vertical axis represents relative the level of ADGRE3 protein encoded by mRNA, and the calculation of nADGRE3 has been described in the summary of the invention.

The significance of differences in mRNA or protein expression levels between groups is indicated by "ns" or several "*" symbols:
"ns" means no significant difference (p > 0.05);
"*" means significant difference (p < 0.05);
"**" means highly significant difference (p < 0.01);
"***" means very highly significant difference (p < 0.001);
"****" means extremely significant difference (p < 0.0001).

### DETAILED DESCRIPTION OF THE INVENTION

Further explanations of the structures involved in the invention, or the technical terms used herein are provided below. Unless otherwise specified, the terms are interpreted according to their common meanings in the relevant field.

### Diagnosis

In this invention, "diagnosis" refers to an assessment concept regarding health status. It includes predicting whether an individual is healthy or unhealthy, as well as evaluating the treatment effectiveness during the disease course. Therefore, the diagnosis of this invention at least includes one or more of the following aspects:
1. Diagnosing whether an individual has systemic inflammation or a specific type of systemic inflammation, including: distinguishing between healthy individuals and systemic inflammation patients, distinguishing patients with local inflammation or non-inflammatory diseases from systemic inflammation patients, and also distinguishing systemic inflammation caused by different etiologies (such as distinguishing bacterial systemic inflammation from non-bacterial systemic inflammation, or viral from bacterial), etc.; or
2. Predicting the risk that an individual may develop systemic inflammation or a specific type of systemic inflammation, including: predicting the risk of systemic inflammation in individuals without systemic inflammation, predicting the risk of local inflammation progressing to systemic inflammation, predicting the risk of systemic inflammation in patients with chronic diseases, etc.; or
3. Monitoring the disease progression of systemic inflammation patients, judging whether the condition of systemic inflammation patients is worsening or improving, assessing the risk of systemic inflammation worsening into sepsis; or prognosis evaluation of systemic inflammation patients, determining the disease progression trend, thereby predicting the risk of death for systemic inflammation patients; or
4. Providing drug guidance for patients, including: intervening with medication to prevent the development of systemic inflammation when a non-systemic inflammation patient shows a trend toward systemic inflammation; increasing drug dosage to improve treatment efficacy when systemic inflammation worsens; reducing drug dosage to minimize adverse drug reactions or drug resistance when the patient improves; the drugs involved can be any medication for treating systemic inflammation, including but not limited to antibiotics, antiviral drugs, and hormone drugs.

### ADGRE3 mRNA or the Encoded Protein

The ADGRE3 gene (gene ID ENSG00000131355) is located on chromosome 19 and has been identified as a protein-coding gene. The gene structure consists of multiple exons, and alternative splicing produces multiple transcript variants encoding different isoforms. The expression profile of ADGRE3 indicates its role in phagocyte function, intercellular interactions, and/or signal transduction during inflammatory processes. This gene is primarily expressed in immune system cells, with higher expression in synovial granulocytes than in peripheral blood granulocytes, which may be due to the inflammatory microenvironment in synovial fluid.

The ADGRE3 protein is a G protein-coupled receptor (GPCR) that may play a role in myeloid-myeloid interactions during immune and inflammatory responses. A soluble form of the receptor's ligand exists on the surface of monocyte-derived macrophages and activated neutrophils. Diseases associated with the ADGRE3 gene include inflammatory bowel disease, possibly related to its role in immune and inflammatory responses. ADGRE3 protein is involved in multiple pathways, including innate immunity and downstream GPCR signaling. The protein product of this gene interacts with other proteins in the immune system. ADGRE3 protein is expressed in various tissues, including bone marrow and lymphoid tissue, with a tissue-enhanced expression pattern. The protein is expected to be secreted, with some isoforms located on the membrane.

In summary, ADGRE3 is a protein-coding gene that plays a crucial role in the immune system, especially in phagocyte function, intercellular interactions, and signal transduction during inflammation. Its expression is mainly in immune cells and involves multiple pathways related to immune and inflammatory responses.

The ADGRE3 gene has multiple mRNA transcript variants, and any one of these mRNAs or their fragments can serve as a biomarker in this invention. One principal mRNA transcript sequence is NM\_032571.5. The mRNA targets of this invention include the following six sequences: XM\_054322398.1, XM\_047439546.1, XM\_011528374.3, NM\_001289158.2, NM\_001289159.2, and NM\_032571.5. These transcript sequences are provided by the RefSeq database. It is understood that any method capable of specifically detecting the quantity or copy number of these mRNAs can be used in the diagnostic methods of this invention, such as conventional thermal cycling amplification (PCR), isothermal amplification, or gene editing techniques for nucleic acid quantification, as well as any compatible reagents. When amplification is needed, designing suitable primers is straightforward.

Because the ADGRE3 gene has multiple mRNA transcript variants, the proteins translated from different mRNAs may have different amino acid sequences. Therefore, any ADGRE3 protein translated from any transcript variant, or their fragments can serve as a biomarker in this invention. It is understood that any method capable of specifically detecting the amount of ADGRE3 protein can be used in the diagnostic methods of this invention, such as flow cytometry, Coomassie brilliant blue staining, or any protein detection methods and compatible reagents for testing or detection.

An amino acid sequence of a human ADGRE3 protein (SEQ ID NO:1):

MQGPLLLPGLCFLLSLFGAVTQKTKTSCAKCPPNASCVNNTHCTCNHGYTSGSGQ
KLFTFPLETCNDINECTPPYSVYCGFNAVCYNVEGSFYCQCVPGYRLHSGNEQFSNSN
ENTCQDTTSSKTTEGRKELQKIVDKFESLLTNQTLWRTEGRQEISSTATTILRDVESKV
LETALKDPEQKVLKIQNDSVAIETQAITDNCSEERKTFNLNVQMNSMDIRCSDIIQGDT
QGPSAIAFISYSSLGNIINATFFEEMDKKDQVYLNSQVVSAAIGPKRNVSLSKSVTLTF
QHVKMTPSTKKVFCVYWKSTGQGSQWSRDGCFLIHVNKSHTMCNCSHLSSFAVLMA
LTSQEEDPVLTVITYVGLSVSLLCLLLAALTFLLCKAIRNTSTSLHLQLSLCLFLAHLLF
LVGIDRTEPKVLCSIIAGALHYLYLAAFTWMLLEGVHLFLTARNLTVVNYSSINRLMK
WIMFPVGYGVPAVTVAISAASWPHLYGTADRCWLHLDQGFMWSFLGPVCAIFSANLV
LFILVFWILKRKLSSLNSEVSTIQNTRMLAFKATAQLFILGCTWCLGLLQVGPAAQVM
AYLFTIINSLQGFFIFLVYCLLSQQVQKQYQKWFREIVKSKSESETYTLSSKMGPDSKP
SEGDVFPGQVKRKY

Based on the characteristics of different segments or regions, the human ADGRE3 protein having the amino acid sequence shown in SEQ ID NO:1 can be divided into the following multiple segments or regions:

| **Feature** | **Segment Position** | **Description** |
|---|---|---|
| Signal peptide sequence | 1-21 | / |
| Mature protein sequence | 22-652 | / |
| Topological domain | 22-357 | Extracellular segment |
| Transmembrane region | 358-378 | α-helical transmembrane region |
| Topological domain | 379-389 | Cytoplasmic segment |
| Transmembrane region | 390-410 | α-helical transmembrane region |
| Topological domain | 411-416 | Extracellular segment |
| Transmembrane region | 417-437 | α-helical transmembrane region |
| Topological domain | 438-464 | Cytoplasmic segment |
| Transmembrane region | 465-485 | α-helical transmembrane region |
| Topological domain | 486-508 | Extracellular segment |
| Transmembrane region | 509-529 | α-helical transmembrane region |
| Topological domain | 530-557 | Cytoplasmic segment |
| Transmembrane region | 558-578 | α-helical transmembrane region |
| Topological domain | 579-580 | Extracellular segment |
| Transmembrane region | 581-601 | α-helical transmembrane region |
| Topological domain | 602-652 | Cytoplasmic segment |
| Functional domain | 24-66 | Epidermal growth factor (EGF)-like domain |
| Functional domain | 67-118 | EGF-like domain capable of binding calcium ions |
| Functional domain | 301-350 | GPS domain |
| Region | 621-652 | Disordered or unstructured region |

| | | |
|---|---|---|
| **Feature (FT)** refers to different regions and domains within a protein sequence. Signal peptide sequence (FT SIGNAL): A short amino acid sequence that guides the newly synthesized protein into the endoplasmic reticulum-Golgi pathway, facilitating protein secretion or localization; mature protein sequence (FT CHAIN): after entering the secretory pathway, the signal peptide is recognized and cleaved by a specific signal peptidase. The remaining portion is the mature protein sequence, which typically includes the functional domains and other essential regions that determine the protein's final conformation and biological function; transmembrane region (FT TRANSMEM): refers to hydrophobic α-helical or β-sheet folded segments embedded within the cell membrane. These secondary structures are commonly found in proteins and enable them to span the lipid bilayer; t topological domain (FT TOPO\_DOM): describes the orientation and location of protein segments in relation to the membrane, such as intracellular, extracellular, or transmembrane regions; functional domain (FT DOMAIN): an independently folded structural unit of the protein with specific biological functions. Region (FT REGION): Refers to segments that share certain common features but have unclear or undefined functions, such as low-complexity regions or repeats. | | |

Fragment positions indicate the location of the first and last amino acids of each segment or region within the full-length amino acid sequence of the protein.

Extracellular segments: Protein regions located outside the cell membrane. Helical (α-helix) regions: Regions of the protein that adopt α-helical structures, a common secondary structure with a coiled arrangement. Many transmembrane proteins contain multiple α-helical segments that insert into the lipid bilayer, forming transmembrane domains. Cytoplasmic segments: Protein regions located inside the cell (cytoplasm), often containing domains or regulatory regions involved in intracellular signal transduction and interactions with intracellular effector molecules. EGF-like domains: Independently folded modules found in various proteins, often mediating protein-protein interactions. EGF-like domains usually contain six conserved cysteine residues forming three disulfide bonds (1-3, 2-4, 5-6). Some EGF-like domains have calcium-binding capability. GPS domain: A conserved region approximately 40 amino acids in length, rich in canonical cysteine and tryptophan residues. It is among the most conserved components of G protein-coupled receptors (GPCRs). Disordered or unstructured regions: These lack a fixed three-dimensional structure and exhibit high conformational flexibility and dynamics. Such regions are typically rich in polar and charged residues and play crucial roles in protein folding, interactions, and regulation.

It is understood that the ADGRE3 protein sequences in mammals may differ from the human version by a few amino acids. Thus, ADGRE3 proteins, protein fragments, amino acids, or amino acid fragments (peptides) from mammals can also be used as biomarkers for detection or testing in the present invention.

In subsequent descriptions of this invention, unless specifically indicated otherwise, the term "gene" refers to an mRNA or mRNA fragment, while "protein" refers to a full-length protein or protein fragment, and can also include full-length peptides or peptide fragments, or full-length amino acids or amino acid fragments. The term "fragment" refers to shorter sequences relative to the full-length, such as gene or amino acid sequences, for example, extracellular protein segments or peptide fragments.

### Leukocytes and Neutrophils

Leukocytes (white blood cells) are a class of colorless, spherical, nucleated blood cells. They are not a homogeneous cell population and can be categorized into three main types based on their morphology, function, and origin: granulocytes, monocytes, and lymphocytes. Granulocytes can be further subdivided into neutrophils, eosinophils, and basophils according to the staining properties of their cytoplasmic granules.

Leukocytes typically possess active motility, allowing them to migrate from within blood vessels to extravascular tissues, or from tissues back into the bloodstream. Therefore, leukocytes are found not only in the blood and lymph but are also widely distributed in tissues outside of blood and lymphatic vessels. Most leukocytes only transiently circulate in the blood before migrating into tissues where they perform their functions.

Neutrophils are a type of leukocyte whose primary role is to defend against microbial invasion. They possess the ability to deform and perform phagocytosis and play a critical role in host defense and disease resistance. In healthy individuals, neutrophils are usually present in the bloodstream; however, in patients with disease-particularly those with systemic inflammation, leukocytes rapidly infiltrate the sites of inflammation. As a result, leukocytes, including neutrophils, are often found in various tissues of such patients, and can therefore be detected in different types of clinical samples.

In the present invention, when ADGRE3 mRNA is used as a biomarker, detection is generally based on the transcription level in leukocytes. For example, a transcription level lower than normal may indicate a risk of developing systemic inflammation. It is understood that the protein translated from this mRNA can also serve as a biomarker, and we have found that using the protein as a biomarker offers higher sensitivity and specificity. Thus, in theory, proteins derived from any type of cell within the leukocyte population may be used, including granulocytes, monocytes, or lymphocytes, as well as any subtype of granulocytes such as neutrophils, eosinophils, or basophils. Although the amount of protein translated varies among different cell types, using the same cell type for testing and comparison still provides diagnostic value. For example, comparing protein expression in monocytes from healthy individuals and those with localized or systemic inflammation shows significant differences. Of course, in some embodiments, using neutrophil-derived protein for testing is also applicable.

### ADGRE3 mRNA or ADGRE3 Protein as a Biomarker

The present invention has found that the mRNA transcribed from the ADGRE3 gene shows a decreasing trend in peripheral blood leukocytes as inflammation progresses, especially during the transition from localized inflammation to systemic inflammation, where it shows a significant decline. Furthermore, even among different types of systemic inflammatory conditions caused by various factors, there are notable differences in expression levels.

Initially, in an effort to identify genes playing a critical role in sepsis pathogenesis, our team analyzed and compared gene expression profiles in peripheral blood from sepsis patients and patients with non-infectious systemic inflammation using public databases. We found that expression of the ADGRE3 gene in peripheral blood was significantly altered in sepsis patients (Figure 1). As shown in Figure 1, among the two most significantly downregulated genes, ADGRE3 exhibited a marked decrease, whereas the other gene, Membrane Metalloendopeptidase (MME), although downregulated, was not suitable as a biomarker for systemic inflammation. This is because additional analyses using data cohorts of infectious diseases and sepsis revealed that MME expression levels did not differ significantly between sepsis patients and healthy controls. Therefore, it is evident from Figure 1 that the downregulation of ADGRE3 can serve as a biomarker to distinguish sepsis patients from those with non-infectious systemic inflammatory conditions. Naturally, the corresponding protein can also be used as a biomarker for this purpose.

Next, we analyzed whether there was a difference in ADGRE3 expression between sepsis patients and healthy individuals. We again found significant downregulation of this gene (Figure 2). This indicates that the gene can also distinguish sepsis patients from healthy individuals. Although many genes were downregulated in Figure 2, not all of them are suitable as biomarkers, and some are already known to be downregulated.

In subsequent experiments, we focused specifically on this gene and its protein. We analyzed expression changes of ADGRE3 in various types of infections using public datasets, and collected patient samples from our hospital to validate mRNA and protein expression. When compared with existing clinical inflammatory markers, we found that this marker exhibited extremely high sensitivity in indicating systemic inflammation-surpassing current clinically used indicators, which have not significantly improved the sensitivity or specificity of inflammatory disease diagnosis.

We further discovered that ADGRE3 mRNA transcription levels in the peripheral blood of patients with systemic inflammation due to bacterial or viral infections were significantly lower than in healthy controls. This trend was consistently observed in infections caused by Gram-positive bacteria, H1N1, dengue virus, COVID-19, RSV, and influenza virus. These findings validate the effectiveness of using ADGRE3 mRNA to diagnose systemic inflammation due to infectious diseases. Moreover, we also found that ADGRE3 mRNA levels could distinguish between Gram-positive bacterial infection and H1N1 infection, asymptomatic and symptomatic COVID-19 patients, as well as acutely infected and recovered RSV patients.

Therefore, ADGRE3 mRNA or protein can not only serve as a diagnostic marker for systemic inflammation caused by infections, but also help to pinpoint the source of the infection. This enables more targeted treatment and better monitoring of disease progression.

Furthermore, clinical validation in healthy individuals, patients with non-infectious systemic inflammatory response syndrome (SIRS), and patients with sepsis showed that ADGRE3 mRNA or protein levels in leukocytes were significantly different across these three groups. Thus, ADGRE3 mRNA or protein can serve as a biomarker to differentiate and diagnose SIRS and sepsis. Diagnostic results from the validation cohort showed that the area under the curve (AUC) for distinguishing healthy individuals from sepsis patients was 0.920, the AUC for distinguishing sepsis from SIRS was 0.864, and the overall AUC for diagnosing sepsis across all sample sets was 0.862. These results indicate that ADGRE3 mRNA transcription levels in leukocytes can accurately diagnose sepsis.

### Inflammation

Inflammation is a physiological response of the body to injury, infection, or irritation, aimed at eliminating pathogens, repairing tissue, and restoring tissue function. Inflammation typically presents with local symptoms such as redness, swelling, heat, pain, and functional impairment.

The process of inflammation generally includes the following steps:
Inflammatory triggers: These can be external or internal factors such as infection, trauma, chemical stimuli, or allergic reactions.
Release of inflammatory mediators: Damaged cells release mediators such as histamine, prostaglandins, and cytokines, which cause vasodilation, increased vascular permeability, and leukocyte infiltration.
Leukocyte infiltration: White blood cells, especially neutrophils, migrate through the vascular wall into tissues to eliminate pathogens and cellular debris.
Tissue repair: During inflammation, the body initiates tissue repair mechanisms, including fibrin deposition, cell proliferation, and tissue regeneration.

The treatment of inflammation depends on its cause and severity. Common treatment methods include:
Nonsteroidal anti-inflammatory drugs (NSAIDs): Such as ibuprofen and acetaminophen, used to relieve pain and reduce inflammation.
Corticosteroids: Such as prednisone, used to control the inflammatory response.
Immunosuppressants: Used to suppress overactive immune responses.
Antibiotics: Used to treat inflammatory conditions caused by infections.
Physical therapy: Such as cold or heat compresses, which can help alleviate inflammation and pain.

### Systemic Inflammation

Systemic inflammation (also referred to as systemic inflammatory response) is a widespread inflammatory reaction that is not confined to a specific tissue or organ, but rather involves the entire body. This type of response is usually a nonspecific reaction of the body to infections or inflammatory triggers, such as bacteria, fungi, viruses, trauma, burns, or surgery. In contrast to localized, tissue- or organ-specific inflammation (which is specific), systemic inflammation represents a more severe stage of the inflammatory process and may develop from a localized inflammation.

In one embodiment of the present invention, systemic inflammation is defined as a state in which 100% of the body's tissues or organs exhibit inflammatory responses. However, this invention also covers conditions where inflammation occurs in more than 50% but less than 100% of tissues or organs. From another perspective, the transition from localized (specific) inflammation to nonspecific systemic inflammatory response is also encompassed by this invention. All the above conditions fall within the scope of the term "**systemic inflammation**." For example, when localized inflammation occurs, the marker described in this invention can be used to monitor disease progression. At this stage, the biomarker level may not show a significant decrease. However, once a significant reduction compared to previous measurements is detected, systemic inflammation can be diagnosed. Alternatively, if a persistent downward trend in the biomarker level is observed, it may indicate the potential progression of localized inflammation into systemic inflammation. This allows for early intervention and treatment to prevent or mitigate systemic inflammation.

Characteristics of Systemic Inflammatory Response include: Release of inflammatory mediators: During systemic inflammation, the body releases various mediators such as cytokines, chemokines, and interleukins, which drive both local and systemic inflammatory responses. Systemic symptoms: Patients may exhibit systemic symptoms such as fever, chills, generalized fatigue, tachycardia, rapid breathing, and either elevated or reduced peripheral white blood cell counts. Organ dysfunction: In severe cases, the excessive release of inflammatory mediators may lead to multiple organ dysfunction syndrome (MODS), posing a serious threat to life. Immune dysregulation: The immune system may become hyperactive during systemic inflammation, leading to immune imbalance, and potentially triggering autoimmune responses. Life-threatening conditions: In severe cases, such as those caused by serious infections or bacterial toxins, systemic inflammation may progress to severe sepsis or septic shock, which can be life-threatening.

Managing systemic inflammation requires early recognition and active intervention. The goals of treatment include controlling the inflammatory response, preserving organ function, providing symptomatic support, and treating the underlying cause. Common clinical approaches include antibiotic therapy, hemodynamic support, and immunomodulatory treatments. Early intervention can significantly reduce the severity of systemic inflammation and improve patient survival and outcomes.

### Distinction Between Systemic and Non-Systemic Inflammation

From a medical perspective: When distinguishing systemic inflammation from non-systemic inflammation, the following aspects should be considered:
1. Medical history and clinical presentation: Systemic inflammation: Patients may exhibit systemic symptoms such as fever, fatigue, general malaise, and inflammation may involve multiple organ systems. Systemic inflammation is often accompanied by elevated systemic inflammatory markers. Non-systemic inflammation: Inflammation may be localized to a specific site or organ, manifesting as localized symptoms such as redness, swelling, pain, and impaired function at the affected area.
2. Laboratory tests: Systemic inflammation: Often associated with abnormalities in complete blood counts, increased inflammatory markers (e.g., C-reactive protein, procalcitonin), and elevated systemic inflammatory mediators. Non-systemic inflammation: Laboratory results may show inflammation limited to the affected site, and blood counts and inflammatory markers may not be as elevated as in systemic inflammation.
3. Imaging studies: Systemic inflammation: Imaging may reveal inflammatory involvement in multiple organ systems, such as multi-organ infection foci during systemic inflammation. Non-systemic inflammation: Imaging findings usually show inflammatory changes limited to the affected local site, such as localized infection or inflammation.
4. Pathogen detection: Systemic inflammation: Pathogens may be detected in multiple body sites via blood cultures or other diagnostic methods. Non-systemic inflammation: Pathogen detection may be positive only at the local or specific affected site.
5. Treatment response: Systemic inflammation: Treatment may require systemic antibiotics or immunomodulatory therapy. Non-systemic inflammation: Local treatments (e.g., topical antibiotics or anti-inflammatory agents) are often effective for controlling inflammation. By integrating medical history, clinical presentation, laboratory and imaging findings, pathogen detection, and treatment response, infectious disease specialists can more accurately distinguish systemic inflammation from non-systemic inflammation and develop appropriate treatment plans.

From an immunological perspective: When distinguishing systemic inflammation from non-systemic inflammation, consider the following:
1. Scope of immune response: Systemic inflammation: Involves abnormal or dysregulated immune activity throughout the body, causing generalized immune responses affecting multiple organs and tissues. Immune cells and inflammatory mediators may be active in multiple body sites. Non-systemic inflammation: Immune response is localized to specific organs or sites; inflammation is mainly confined to the affected local area.
2. Immune cell involvement: Systemic inflammation: Involves abnormal activity of various immune cell types, including T lymphocytes, B lymphocytes, neutrophils, macrophages, etc., leading to systemic immune and inflammatory responses. Non-systemic inflammation: Immune cell activity is primarily limited to the affected site, such as specific tissues or organs.
3. Release of inflammatory mediators: Systemic inflammation: Increased release of inflammatory mediators across the whole body, potentially causing systemic symptoms and elevated inflammatory markers. Non-systemic inflammation: Release of inflammatory mediators is mainly confined to the local area, and systemic inflammatory markers may not rise significantly.
4. Immune regulation mechanisms: Systemic inflammation: May involve broad immune system regulation, requiring comprehensive modulation of immune cell activity and inflammatory mediator release. Non-systemic inflammation: Treatment often focuses on local control of inflammation without disrupting systemic immune balance.

By comprehensively considering the range of immune responses, immune cell participation, inflammatory mediator release, and immune regulatory mechanisms, immunologists can more accurately differentiate systemic from non-systemic inflammation and develop appropriate immunomodulatory therapies.

Any localized inflammation or systemic inflammation as defined above falls within the scope of the present invention. The biomarkers of this invention can not only distinguish localized inflammation from systemic inflammation but also differentiate systemic inflammation from healthy individuals.

### Infectious Inflammation

Infectious inflammation can develop from a local infection into systemic inflammation. Infectious inflammation is an inflammatory response caused by infection. Infection refers to the invasion and proliferation of pathogens (such as bacteria, viruses, fungi, or parasites) in the host organism. When the body is infected, the immune system initiates an inflammatory response to eliminate the pathogens and repair damaged tissues.

Characteristics of infectious inflammation include:
Pathogen invasion: Infectious inflammation is caused by the invasion of pathogens, which can be bacteria, viruses, fungi, or parasites. Inflammatory response: The infection-triggered inflammation is a natural immune defense mechanism involving the release of inflammatory mediators, activation, and migration of white blood cells.
Local symptoms: Infectious inflammation often manifests as local symptoms such as redness, swelling, pain, heat, and functional impairment. These symptoms are part of the body's response to fight infection. Systemic inflammation: Severe infections may cause systemic symptoms including fever, chills, fatigue, headache, nausea, and vomiting.
Severe complications: In serious cases, infection may lead to sepsis, septic shock, multiple organ dysfunction syndrome (MODS), and can be life-threatening. These conditions fall under systemic inflammation with a classification based on severity.

The key to managing infectious inflammation lies in early identification and prompt treatment of the infection. Treatment methods include antibiotics (for bacterial infections), antiviral drugs (for viral infections), antifungal drugs (for fungal infections), alongside supportive therapies to maintain vital signs stability. Prevention involves maintaining good personal hygiene, vaccination, avoiding contact with known infection sources, and timely treatment of infections. Populations at higher risk (such as immunocompromised individuals or patients with chronic diseases) require close attention to infection prevention measures.

### Non-infectious Inflammation

Non-infectious inflammation can also progress from local to systemic inflammation. Non-infectious inflammation refers to inflammatory responses not caused by external pathogens (such as bacteria, viruses, fungi, or parasites). This type of inflammation may be triggered by abnormal immune responses, transplant organ rejection, tissue injury, chemical irritation, or other non-infectious factors. Common non-infectious inflammatory diseases include:
Autoimmune diseases: Such as rheumatoid arthritis, systemic lupus erythematosus, vasculitis, autoimmune hepatitis, autoimmune thyroiditis (Hashimoto's thyroiditis and Graves' disease), multiple sclerosis, Sjögren's syndrome, and transplant rejection. These diseases result from the immune system mistakenly attacking the body's own tissues.
Inflammatory bowel diseases: Such as Crohn's disease and ulcerative colitis, caused by chronic inflammation in the intestines.
Allergic reactions: Allergic diseases like asthma and allergic rhinitis result from abnormal immune responses to allergens.
Metabolic diseases: For example, diabetes and obesity, which are closely associated with inflammatory responses.
Inflammation caused by tissue injury: Trauma, chemical irritation, and radiation damage may all trigger non-infectious inflammation.

Non-infectious inflammation differs in etiology and pathogenesis from infectious inflammation but can share some clinical manifestations, such as fever, pain, redness, and swelling.

Management of non-infectious inflammation typically involves controlling the inflammatory response, relieving symptoms, and reducing tissue damage. Treatment depends on the disease type and severity. For autoimmune diseases, standard treatments include immunosuppressants and anti-inflammatory drugs. Other non-infectious inflammations may be treated with medications, physical therapies, or surgery. Timely diagnosis and treatment of non-infectious inflammation help alleviate symptoms, control disease progression, and improve patients' quality of life.

### Sepsis

Sepsis is a form of systemic inflammatory response syndrome (SIRS), representing a more severe type of systemic inflammation commonly seen in patients with severe trauma or infectious diseases. The etiology includes infections caused by bacteria, fungi, viruses, and parasites, which lead to an imbalance of the body's inflammatory response and immune regulation. Sepsis can progress to severe sepsis and septic shock, resulting in organ dysfunction and circulatory failure, which can be life-threatening.

Sepsis refers to a confirmed or suspected infection that triggers systemic inflammatory response syndrome. Severe sepsis refers to sepsis accompanied by organ dysfunction and tissue hypoperfusion caused thereby. Septic shock is sepsis with associated hypotension that cannot be reversed by fluid resuscitation.

### Diagnostic criteria for sepsis:

Presence of a confirmed or suspected infection along with one or more of the following clinical features: Signs and clinical symptoms: Temperature > 38.3°C or < 36°C; Heart rate > 90 beats per minute, or exceeding two standard deviations above the normal range for age; Tachypnea: respiratory rate > 20 breaths per minute or hyperventilation, PaCO2 < 32 mmHg.

Altered mental status: Significant edema or positive fluid balance exceeding 20 ml/kg in 24 hours; Hyperglycemia without a history of diabetes; Hemodynamics: Hypotension: systolic blood pressure < 90 mmHg, mean arterial pressure (MAP) < 70 mmHg, or adult systolic blood pressure drop > 40 mmHg or two standard deviations below normal for age group.

Laboratory tests: Markers of inflammatory response: leukocytosis (WBC > 12,000/µl), leukopenia (WBC < 4,000/µl), normal WBC but immature forms > 10%, plasma C-reactive protein (CRP) elevated beyond two standard deviations of normal, plasma procalcitonin elevated beyond two standard deviations of normal. Indicators of organ dysfunction: hypoxemia (PaO2/FiO2 < 300 mmHg), acute oliguria despite adequate fluid resuscitation, serum creatinine > 44.2 µmol/L (0.5 mg/dl), coagulation abnormalities, intestinal obstruction (absent bowel sounds), thrombocytopenia (PLT < 10,000/µl), hyperbilirubinemia.

Tissue perfusion indicators: elevated lactate (> 1 mmol/L), decreased capillary refill ability or petechiae formation. SOFA score: For patients with confirmed or suspected infection, an increase of 2 or more points in the Sequential Organ Failure Assessment (SOFA) score from baseline indicates sepsis. Because SOFA scoring is complex, bedside quick SOFA (qSOFA) criteria are often used to identify critically ill patients. If at least 2 of the following 3 criteria are met, combined with organ dysfunction evaluation, sepsis can be preliminarily assessed: Respiratory rate ≥ 22 breaths/min; Altered mental status Systolic blood pressure ≤ 100 mmHg.

Diagnostic criteria for severe sepsis and septic shock: Sepsis accompanied by organ dysfunction and/or tissue hypoperfusion, evidenced by any of the following: Clinical signs: hypotension due to sepsis; urine output < 0.5 ml/kg/h for at least 2 hours despite adequate fluid resuscitation; acute lung injury unrelated to pneumonia with PaO2/FiO2 < 250 mmHg; acute lung injury caused by pneumonia with PaO2/FiO2 < 200 mmHg; Laboratory findings: lactate above normal range; serum creatinine > 176.8 µmol/L (2.0 mg/dl); bilirubin > 34.2 µmol/L (2 mg/dl); platelet count < 100,000/µl; coagulation disorder (international normalized ratio > 1.5).

Currently, treatment of sepsis can be generally divided into three parts: etiological treatment, supportive therapy, and immunomodulatory therapy. Etiological treatment includes early elimination of infectious foci and administration of effective antibiotics. Supportive therapy includes early circulatory resuscitation, mechanical ventilation, renal replacement therapy, metabolic support, anticoagulation, and other measures targeting damage to different organs and systems. Immunomodulatory therapy mainly refers to treatments capable of affecting the body's immune and inflammatory responses.

The content of ADGRE3 mRNA and/or ADGRE3 protein discovered in the present invention can to some extent reflect the etiological type in patients with systemic inflammation. On one hand, this refers to distinguishing between infectious inflammation and non-infectious inflammation from a broad classification perspective, as well as differentiating the severity of systemic inflammatory diseases, such as distinguishing sepsis from septic shock, thereby avoiding the use of the aforementioned complex diagnostic criteria while improving the sensitivity, specificity, and accuracy of diagnosis. On the other hand, it refers to identifying the causative factors of the disease, such as differentiating infectious inflammation caused by different pathogens, thereby assisting in etiological treatment and supportive therapy.

Sepsis has an extremely high mortality rate, but currently, there is no technology available to predict the mortality risk in sepsis patients. The ADGRE3 protein levels discovered in this invention can function to predict the risk of death in patients. Additionally, by monitoring the expression level of ADGRE3 protein in patient neutrophils daily, an accurate assessment of whether the patient's sepsis is improving can be made, thereby assisting clinical treatment.

### Cut-off Value

Definition of Cut-off Value: A cut-off value refers to the numerical threshold used to convert a continuous variable into a discrete variable, serving as the critical point to determine whether a result is positive or negative. It determines the classification of the result.

Setting the Cut-off Value: Based on Reference Range: The normal reference range is an important basis for setting the cut-off. By statistically analyzing samples from healthy populations, the normal reference range is determined and used as a key reference for the cut-off. Based on Disease Characteristics: Understanding the characteristics of the disease being tested is crucial for setting the cut-off. Some diseases may require higher or lower cut-offs for accurate diagnosis. Based on Receiver Operating Characteristic (ROC) Curve: The ROC curve helps evaluate sensitivity and specificity at different cut-offs to find the optimal cut-off for the best diagnostic performance. Based on Clinical Experience: Clinical experience plays a key role in setting the cut-off. Laboratory experts should consider clinicians' needs and real-world conditions, adjusting the cut-off accordingly. Based on Quality Control: Ensuring the cut-off meets quality control standards is necessary to guarantee the accuracy and reliability of results. Based on Standardized Guidelines: Reference to relevant standardized guidelines or recommendations from professional organizations can provide guidance for setting cut-offs. Regular Review and Update: Cut-offs should be regularly reviewed and updated to ensure they remain consistent with the latest clinical practice and data.

By comprehensively considering these factors, clinical laboratory diagnostic experts can establish reasonable cut-offs to ensure the accuracy of laboratory test results and the effectiveness of clinical diagnosis.

### Method of Setting the Cut-off in This Invention:

The invention detects ADGRE3 protein expression in neutrophils from healthy, non-inflammatory populations by flow cytometry. The mean fluorescence intensity (MFI) of ADGRE3 protein is calculated for each sample, and the average MFI in the healthy population is obtained. Under the same conditions, the patient's neutrophil ADGRE3 protein MFI is measured. The expression index of ADGRE3 in patient neutrophils (nADGRE3) is calculated by dividing the patient's neutrophil ADGRE3 MFI by the average ADGRE3 MFI of the healthy controls, that is: nADGRE3 = Patient neutrophil ADGRE3 MFI / Average healthy control neutrophil ADGRE3 MFI.

Therefore, the nADGRE3 value for healthy controls is approximately or equal to 1. Based on this, statistical analysis of sensitivity and specificity of nADGRE3 in infectious and non-infectious inflammatory responses identified the optimal cut-off value as 0.8361. Using this method, if the value is greater than 0.8361, the condition can be considered healthy or non-systemic inflammation (localized inflammation or non-inflammatory disease). If the value is less than 0.8361, it can be considered systemic inflammation. Calculating the neutrophil ADGRE3 protein expression index (nADGRE3) is only one form of cut-off setting and sample classification. Cut-offs and classifications can also be based on ADGRE3 mRNA copy number, ADGRE3 protein quantity (mass or concentration), or any other known method, with the diagnostic classification results being the same.

The above describes one method for setting the cut-off value between healthy populations and systemic inflammation. However, it is understandable that since the comparison targets differ, the cut-off settings also vary. For example, when comparing different severities within systemic inflammation cases, the cut-off is set based on comparisons between different severity levels. To achieve differentiation, the test values within the same category generally need to show statistically significant differences (P < 0.05, or highly significant difference P < 0.01, with at least any corresponding level where P < 0.05 being acceptable as a specific cut-off value). The units depend on the different testing methods used, reflecting the differences in the marker levels or amounts of the invention between comparison groups. The cut-off units can be any unit, such as MFI (mean fluorescence intensity), copy number, protein concentration, or any other indirect measure representing the content in the sample.

Regarding the measurement of ADGRE3 mRNA or ADGRE3 mRNA fragment content and the setting of cut-off values, currently, there is considerable variation among laboratories and testing institutions in detection methods and instruments used. Differences also exist in data processing, especially in standardization algorithms, making it difficult to specify an exact cut-off value. However, the significant difference in ADGRE3 mRNA content between systemic inflammation patients and healthy controls objectively exists. Any skilled person in the field can analyze samples from healthy controls and systemic inflammation patients based on their employed detection methods, instruments, and data analysis algorithms to derive appropriate cut-off values for diagnosing systemic inflammation. All these falls within the scope of the present invention's protection. Similarly, the cut-off values for ADGRE3 protein share the same situation.

Common Laboratory Indicators for Early Diagnosis of Systemic Inflammation, Infection Type Differentiation, Prognosis Assessment, Disease Monitoring, and Antibiotic Guidance

### 1. C-Reactive Protein (CRP):

C-reactive protein (CRP) is a protein synthesized in the liver, which typically increases in levels during systemic inflammatory responses and tissue injury. Elevated CRP reflects a non-specific response of the body to infections, inflammation, tissue damage, malignant tumors, and other conditions.

Clinically, CRP is commonly used as an indicator to assess the degree of inflammation and to monitor disease activity. In infectious diseases, CRP levels usually rise as inflammation persists and gradually decrease once inflammation subsides or treatment is effective. Therefore, CRP helps physicians evaluate infection severity, guide treatment planning, and monitor therapeutic efficacy.

It should be noted that although CRP is a commonly used inflammatory marker, it is not highly specific, since many other conditions, such as autoimmune diseases, cardiovascular diseases, and tumors, can also cause CRP elevation. Therefore, clinical judgment using CRP must be combined with the patient's clinical presentation, other laboratory test results, and imaging studies for comprehensive evaluation.

### 2. Procalcitonin (PCT):

Procalcitonin (PCT) is a precursor protein that typically increases during infections and inflammatory responses. In systemic inflammatory responses and infections, PCT levels usually rise significantly. Clinically, PCT levels are commonly used to assess the severity of infection, guide decisions on antibiotic therapy, and monitor patient treatment outcomes.

PCT elevation may occur earlier than other inflammatory markers in infections and inflammation, thus having value in early diagnosis and prognosis assessment. Clinically, PCT is often used to differentiate bacterial infections from non-bacterial infections, guiding antibiotic use decisions, especially in critically ill patients. It is considered a specific marker for diagnosing bacterial infections.

It is important to note that PCT levels are influenced by various factors, including the type and site of infection, and the patient's immune status. Therefore, clinical decision-making based on PCT requires integration with the patient's clinical manifestations, other laboratory tests, and imaging studies.

### 3. CD64 Infection Index:

The CD64 infection index is a recently discovered specific marker for bacterial infection. CD64 is a cell surface marker usually found on monocytes and neutrophils. Its expression significantly increases during bacterial infections and inflammatory processes, making it a potential marker for bacterial infections.

The CD64 infection index assesses infection severity by measuring the expression level of CD64 on the surface of monocytes or neutrophils. During infection, neutrophil surface CD64 expression typically rises significantly, so an increased CD64 infection index may indicate the presence and severity of bacterial infection.

Clinically, the CD64 infection index is used to help differentiate bacterial infections from non-bacterial infections and to evaluate infection severity. However, it is not intended as a standalone diagnostic tool for infection and must be combined with clinical presentation, other laboratory test results, and imaging studies for comprehensive assessment. Additionally, variability in CD64 infection index results may exist across different laboratories and studies, so cautious interpretation is needed during clinical application.

4. CD169: Also known as Siglec-1, CD169 is a membrane glycoprotein belonging to the Siglec protein family. CD169 is mainly expressed on the surface of dendritic cells and macrophages and specifically binds to glycan structures on extracellular viral particles, promoting viral phagocytosis and clearance. Therefore, CD169 plays an important role in infection detection. Studies have shown that CD169 expression is upregulated after viral infection, indicating that CD169 may serve as an early marker for viral infection. In hepatitis virus and HIV infections, CD169 expression is related to viral transmission and clearance. Thus, detecting CD169 expression levels in viral infections can help assess the extent and severity of the infection.
1. HLA-DR: HLA-DR is an MHC class II molecule of the human leukocyte antigen system, mainly expressed on antigen-presenting cells such as dendritic cells and macrophages, and participates in regulating T cell immune responses. During infectious diseases, HLA-DR expression can change, primarily in two ways:
   Increase: Increased HLA-DR expression usually occurs in the early stages of infection as an immune response to infection. Upon infection, immune cells release pro-inflammatory factors that stimulate antigen-presenting cells to express HLA-DR, thereby initiating T cell immune responses. Increased HLA-DR can serve as a biomarker for early infection diagnosis and assessing severity.
   Decrease: Decreased HLA-DR expression often occurs in the late stages of infection, representing immune suppression. After several days or weeks of infection, prolonged stimulation and activation of immune cells may cause dysfunction of antigen-presenting cells and immune exhaustion. At this stage, HLA-DR expression usually declines, indicating suppressed immune response. Decreased HLA-DR can also be used as a biomarker to evaluate post-infection immunosuppression.

6. Heparin-Binding Protein (HBP): HBP is a granular protein derived from neutrophils, mainly stored in azurophilic granules and to a lesser extent in secretory vesicles. HBP belongs to the serine protease family but lacks protease activity. It is a single-chain protein consisting of 222 amino acids, containing 8 cysteine residues, and has glycosylation sites at aspartic acid residues at positions 100, 114, or 145. Its structure is similar to neutrophil elastase, sharing 45% homology.

HBP has a high affinity for lipopolysaccharide lipid A. Upon neutrophil activation and degranulation, HBP is released. During acute bacterial infections, blood HBP concentrations can significantly increase within 1-2 hours, whereas viral infections cause no or mild increases. HBP also acts as a pathogenic factor and decreases rapidly with effective treatment. Thus, measuring HBP levels in patient blood can assist clinical diagnosis and prediction of acute bacterial infections, assess infection severity, and monitor antibiotic efficacy. HBP is also a specific marker for bacterial infection diagnosis.

7. White Blood Cell Count (WBC): White blood cells are part of the immune system, and their numbers typically increase during inflammatory responses. Elevated WBC count reflects the presence and severity of inflammation.

8. Neutrophil-to-Lymphocyte Ratio (NLR): NLR is the ratio of neutrophil count to lymphocyte count and serves as an indicator of inflammatory response and prognosis. A high NLR is generally associated with poor prognosis.

9. Platelet Count: Platelets also change during inflammatory responses. Low platelet counts may be associated with systemic inflammatory responses and an increased risk of bleeding.

10. Serum Lactate Level: Elevated lactate levels may reflect tissue hypoxia and metabolic disturbance, correlating with severe infection and systemic inflammatory response.

These indicators can be used as references for assessing the prognosis of patients with systemic inflammatory responses but require integration with clinical manifestations, imaging studies, and other comprehensive information for overall evaluation.

Specific biomarkers for diagnosing bacterial inflammation include, but are not limited to, CD64, PCT, and HBP. These biomarkers are specific to bacterial inflammation (inflammatory responses caused by bacterial infections). When an individual experiences bacterial inflammation, the measured levels of CD64, PCT, and HBP increase. Conversely, when the individual has no inflammation or non-bacterial inflammation (e.g., viral inflammation or inflammation caused by autoimmune diseases), the measured levels of CD64, PCT, and HBP do not increase or increase insignificantly. Some patients with non-bacterial inflammation may also show elevated levels of CD64, PCT, and HBP, usually because bacterial infection has occurred secondarily as the non-bacterial inflammation progresses. Therefore, in some approaches, any specific marker used to diagnose bacterial infection can be combined with the biomarkers of the present invention to diagnose and distinguish whether systemic inflammation is caused by bacterial infection. For example, distinguishing systemic inflammation caused by bacterial infection from that caused by viral infection, or distinguishing bacterial infection from non-bacterial infection (such as systemic inflammation caused by viral infection, autoimmune disease, or organ transplant rejection).

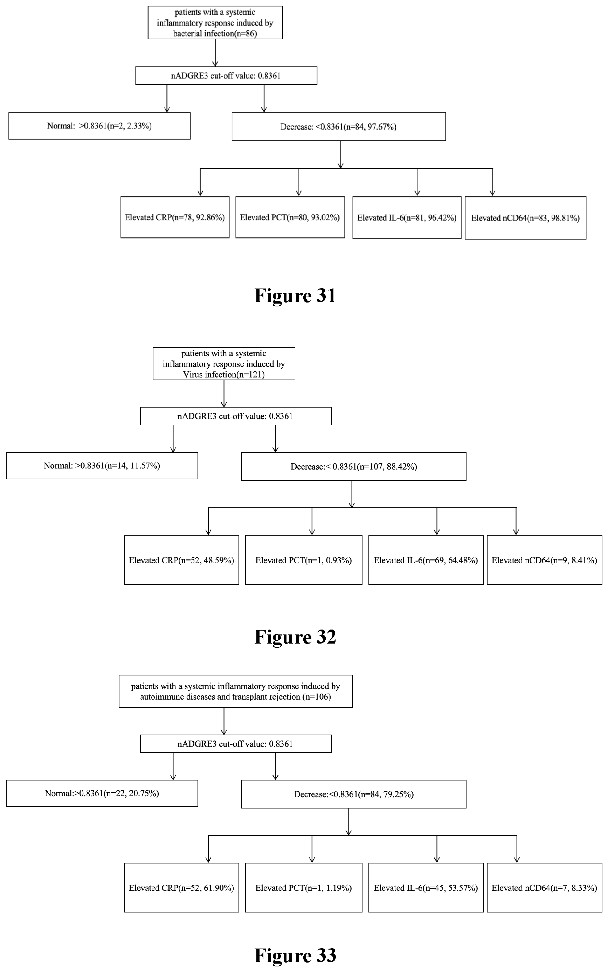

For example, referring to Figure 32, when using flow cytometry and setting 0.8361 as the threshold, our biomarker achieves a detection rate of 88.42% in patients with viral-induced systemic inflammation. Among these detected cases, the specific bacterial infection markers show very low detection rates, indicating that bacterial infection markers, especially specific markers such as PCT and neutrophil CD64 (nCD64), basically show no change (Figure 32). The same situation occurs in systemic inflammation caused by autoimmune diseases or organ transplant rejection (Figure 33). Conversely, in patients with systemic inflammation caused by bacterial infection, the biomarker achieves a detection rate of 97.68%. In these patients, bacterial-specific markers also significantly increase relative to the threshold (PCT and nCD64), with detection rates of 93% and 98%, respectively.

When applying this theory to a blinded clinical experiment, 100 healthy individuals (local infection or healthy population), 100 patients with viral infection-induced systemic inflammation, 100 patients with systemic inflammation caused by autoimmune diseases or organ transplant rejection, and 100 patients with bacterial infection-induced systemic inflammation were randomly mixed and tested. Using the same threshold and flow cytometry to simultaneously test the biomarkers shown in Figure 31, including the protein biomarkers of the present invention, a high concordance with actual conditions was obtained. Among 300 systemic inflammation patients, 295 were detected as having systemic inflammation (detection rate of 98%). Among these, 90 patients showed elevated PCT and CD64, indicating that 90 patients had bacterial infection-induced systemic inflammation, while the other 205 patients did not show significant changes in PCT and CD64 levels. It is therefore understood that combining the biomarkers of the present invention with some currently specific bacterial infection markers can distinguish bacterial infection from non-bacterial infection in systemic inflammation patients. Non-bacterial infection includes viral infection, autoimmune disease, or systemic inflammation caused by organ transplantation. After this differentiation, other clinical indicators or biomarkers can be used to further distinguish viral infection, autoimmune disease, or organ transplant-related systemic inflammation.

Therefore, the present invention finds that nADGRE3 can be combined with bacterial inflammation diagnostic markers to classify or localize systemic inflammation patients. When a known patient's nADGRE3 is decreased, if the bacterial inflammation diagnostic markers show an increase or decrease relative to healthy controls, the patient is diagnosed with bacterial systemic inflammation; otherwise, the patient is diagnosed with non-bacterial systemic inflammation. Further classification (into viral systemic inflammation or non-infectious systemic inflammation) can be performed based on clinical information, medical history, and other laboratory tests.

### Diagnosis and Testing

The terms "diagnosis" or "detection" in the present invention refer to the measurement or assay of biomarkers in a sample, or the quantification of target biomarkers, such as absolute or relative amounts. The presence or quantity of the target biomarker is then used to indicate whether the individual providing the sample may have or is likely to have a certain disease or condition. Here, the terms diagnosis and detection can be used interchangeably.

### Relationship Between Biomarkers and Disease

"Marker" or "biomarker" have the same meaning in the present invention. The relationship means that the presence or change in the level of a certain biomarker in a sample is directly associated with a specific disease-for example, a relative increase or decrease in level indicating whether the individual is a healthy person, has localized infection, or systemic inflammation. It also involves classification of systemic inflammation and the causes of systemic inflammation, such as distinguishing infectious systemic inflammation from non-infectious systemic inflammation.

### Reagents for Testing

Any reagent or method capable of detecting ADGRE3 mRNA or protein can be applied in the present invention. For example, nucleic acid amplification reagents, in situ hybridization, nucleic acid-protein fusion technologies, gene editing technologies, etc., can all be used to detect the expression level or quantity of the ADGRE3 gene. Reagents for protein testing include any reagents capable of detecting the protein encoded by this gene, such as antibody-based reagents. Such antibodies can specifically bind to the protein or protein fragments. In some embodiments, the antibody specifically binds to the extracellular domain or fragment thereof of the protein, or to an extracellular amino acid sequence or peptide fragment. Testing methods can include lateral flow test strips, direct liquid chromatography-mass spectrometry (LC-MS), flow cytometry, ELISA, or other immunoassay principles.

### Antibodies

The antibodies used in the present invention can be any antibodies that specifically bind the analyte in the sample. The antibodies used for binding can be any antibodies known to those skilled in the art. "Antibody" refers to immunoglobulin molecules and antigen-binding fragments thereof that contain antigen-binding sites which specifically bind the analyte, analyte analogs, or ligands ("immune reaction"). This term also includes antibody derivatives that retain binding ability and any proteins containing binding domains homologous or largely homologous to immunoglobulin binding domains. These proteins may be derived from natural sources or partially or fully synthetic. An antibody may be monoclonal or polyclonal. It may belong to any immunoglobulin class, including any human or mammalian immunoglobulin types: IgG, IgM, IgA, IgD, IgE.

"Antibody fragments" are derivatives or parts of an antibody smaller than the full-length antibody that retain significant binding sites of the full-length antibody. Examples of antibody fragments include Fab, Fab', F(ab')2, scFv, Fv, dsFv dimers, and Fd fragments, but are not limited to these. Antibody fragments can be generated by any means. For example, antibody fragments can be produced by enzymatic or chemical cleavage of a whole antibody or by recombinant production from genes encoding antibody portions. In other words, antibody fragments can be partially or fully recombinantly produced. They can be any single-chain antibody fragment, containing multiple linked peptide chains connected, for example, by disulfide bonds. Antibody fragments can also be multimolecular complexes. A functional antibody fragment usually contains at least about 50 amino acids, and often at least about 200 amino acids.

Single-chain Fvs (scFvs) are recombinant antibody fragments consisting only of variable light (VL) and variable heavy (VH) chains covalently linked by a peptide chain. Either the VL or VH chain has an amino-terminal region. The length and composition of the peptide linker is variable, designed to bridge the two variable domains without significantly interfering with atomic arrangement. The linker is usually rich in glycine and serine residues, with some glutamate and lysine residues to increase solubility. A "dimer" refers to a dimer of single-chain Fvs, where the monomeric chains are usually shorter than most single-chain Fvs and show a tendency to form dimers.

An "Fv" fragment consists of a VH and a VL domain connected non-covalently. The term "dsFv" refers to an Fv stabilized by an intermolecular disulfide bond between VH and VL domains. An "F(ab')" fragment is essentially the same as that obtained by digesting immunoglobulin (usually IgG) with pepsin at pH 4.0-4.5. This fragment can also be recombinantly produced. A "Fab'" fragment is essentially the same as that obtained by reducing the disulfide bonds linking the two heavy chains in the F(ab') fragment and can also be recombinantly produced. A "Fab" fragment is essentially the same as that obtained by digesting immunoglobulin (usually IgG) with papain and can also be recombinantly produced. The heavy chain fragment of the Fab fragment is known as the Fd fragment.

### Examples

The present invention will be further described in detail below with reference to the accompanying drawings and specific embodiments. The embodiments are provided only to explain the invention and not to limit the scope of the invention. Unless otherwise specified, the experimental methods used in the following embodiments are conventional methods; materials and reagents used, unless otherwise stated, are commercially available reagents and materials.

### Example 1: Discovery of ADGRE3 as a Biomarker for Diagnosing Systemic Inflammation

The present invention initially aimed to identify genes that play an important role in the pathogenesis of sepsis by analyzing and comparing gene expression differences between sepsis patients and non-infectious systemic inflammation patients in public databases. As shown in Figure 1, the ADGRE3 gene was found to be significantly downregulated. It was considered that this gene could serve as a biomarker to distinguish sepsis patients from patients with non-infectious systemic inflammation. Subsequently, gene expression profiles between sepsis patients and healthy control populations were analyzed. As shown in Figure 2, the ADGRE3 gene was significantly downregulated in sepsis patients, with very significant changes in expression.

Then, we singled out the ADGRE3 gene and validated its expression changes across different infection types in public datasets; specific results are shown in Figures 3 to 11.

For example, as shown in Figure 3, we analyzed 16 healthy control samples (healthy control, labeled in the public database as healthy individuals), 23 systemic inflammation patients caused by bacterial infection, and 28 systemic inflammation patients caused by viral infection. We found that the expression of this gene was significantly decreased in systemic inflammation patients caused by either bacterial or viral infection compared to healthy controls, indicating that this gene can be used to distinguish healthy individuals from those with systemic inflammation caused by viral or bacterial infection. The AUC values were: 0.9063 for distinguishing healthy from viral infection (systemic inflammation), and 0.9402 for distinguishing healthy from bacterial infection (systemic inflammation).

As shown in Figure 4, we analyzed 33 healthy control samples, 18 systemic inflammation patients caused by Gram-positive bacterial infection, and 19 systemic inflammation patients caused by H1N1 viral infection. We found that the expression of the ADGRE3 gene was significantly decreased in patients with systemic inflammation caused by either Gram-positive bacterial infection or H1N1 viral infection compared to healthy controls. The distinguishing AUC value was 0.8215. Among them, the expression of ADGRE3 in systemic inflammation patients caused by Gram-positive bacterial infection was significantly lower than in patients with systemic inflammation caused by H1N1 viral infection.

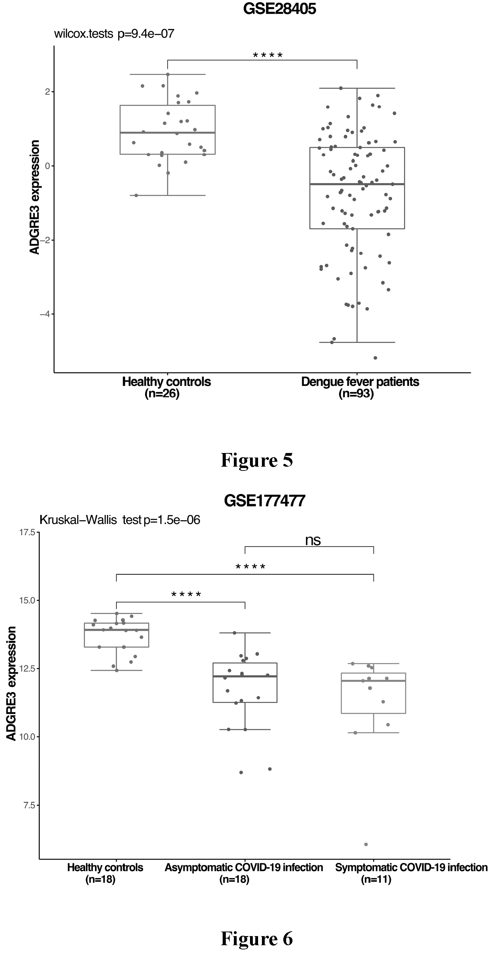

As shown in Figure 5, we analyzed 26 healthy control samples and 93 systemic inflammation patients caused by dengue virus infection. We found that the expression of this gene in systemic inflammation patients caused by dengue virus infection was significantly decreased compared to healthy controls, with an AUC value of 0.8156 for discrimination.

As shown in Figure 6, we analyzed 18 healthy control samples, 18 systemic inflammation patients caused by asymptomatic COVID-19 infection, and 11 symptomatic COVID-19 patients who progressed to systemic inflammation. We found that the expression of the gene was significantly decreased in both asymptomatic and symptomatic systemic inflammation patients relative to healthy controls. The AUC value for distinguishing healthy individuals from COVID-19 infection-induced systemic inflammation was 0.9502. This indicates that the gene expression can confirm systemic inflammation caused by COVID-19 infection regardless of symptom presence. There was no significant difference in gene expression between asymptomatic systemic inflammation and symptomatic systemic inflammation patients.

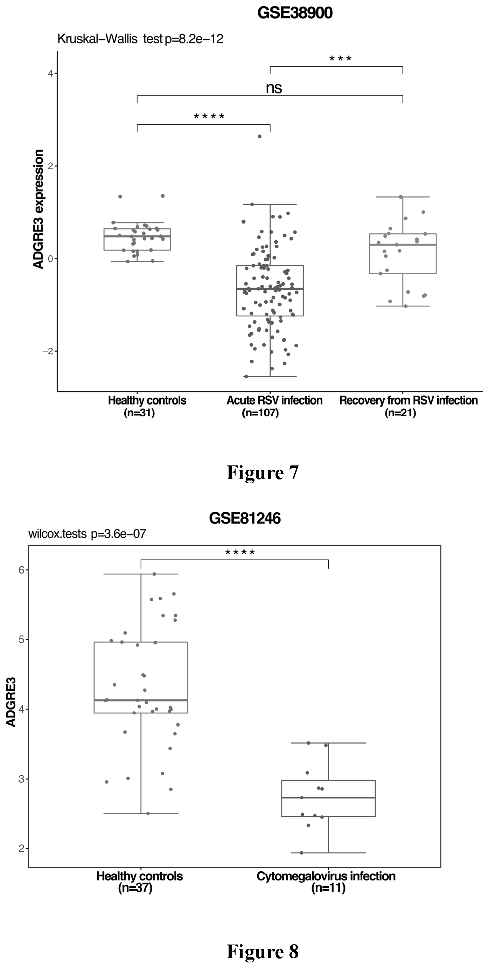

As shown in Figure 7, we analyzed 31 healthy controls and 107 patients with systemic inflammation caused by RSV (respiratory syncytial virus) infection. The gene expression was significantly decreased compared to healthy controls, with an AUC of 0.8936. However, with treatment, gene expression levels in samples transitioning from RSV systemic inflammation to recovery returned to normal healthy levels. This suggests that gene expression can be used to monitor or test the effectiveness of treatment aimed at resolving systemic inflammation.

As shown in Figure 8, we analyzed 37 healthy controls and 11 systemic inflammation patients caused by cytomegalovirus infection. The gene expression was significantly decreased compared to healthy controls, with an AUC value of 0.9459.

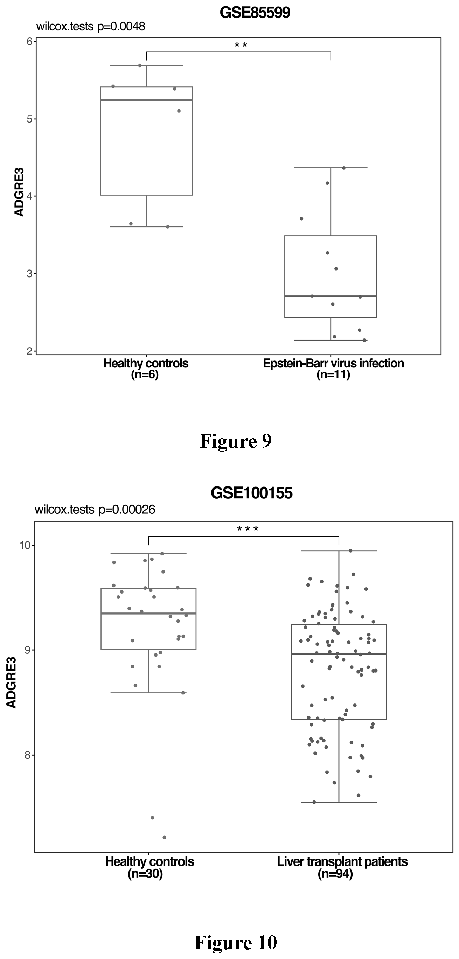

As shown in Figure 9, we analyzed 6 healthy controls and 11 systemic inflammation patients caused by Epstein-Barr virus infection. The gene expression was significantly decreased compared to healthy controls, with an AUC value of 0.9091.

The above viruses, including H1N1, dengue, COVID-19, RSV, cytomegalovirus, and Epstein-Barr virus, are typical viral infections causing systemic inflammation. It can be inferred that the expression of the ADGRE3 gene is significantly decreased in systemic inflammation patients caused by viral infections, and ADGRE3 gene expression can distinguish between healthy individuals and patients with viral infection-induced systemic inflammation.

As shown in Figure 10, we analyzed 30 healthy controls and 94 systemic inflammation patients caused by liver transplant rejection. We found that the gene expression was significantly decreased in patients with systemic inflammation caused by liver transplant rejection, with an AUC value of 0.7220.

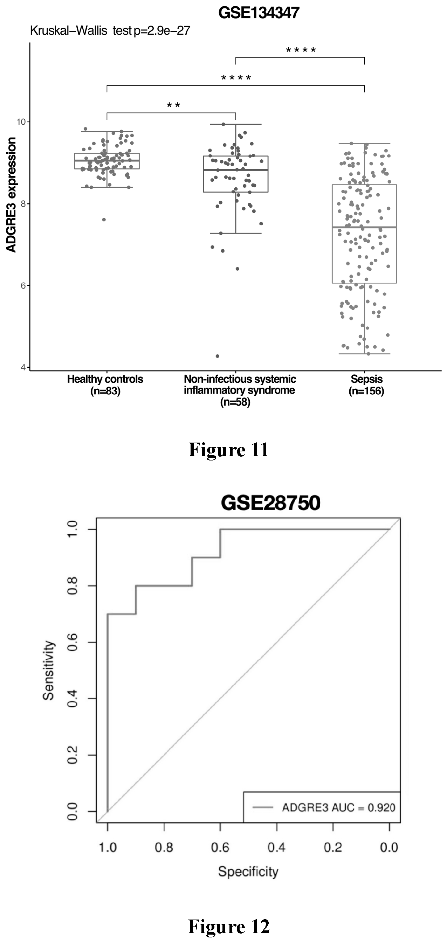

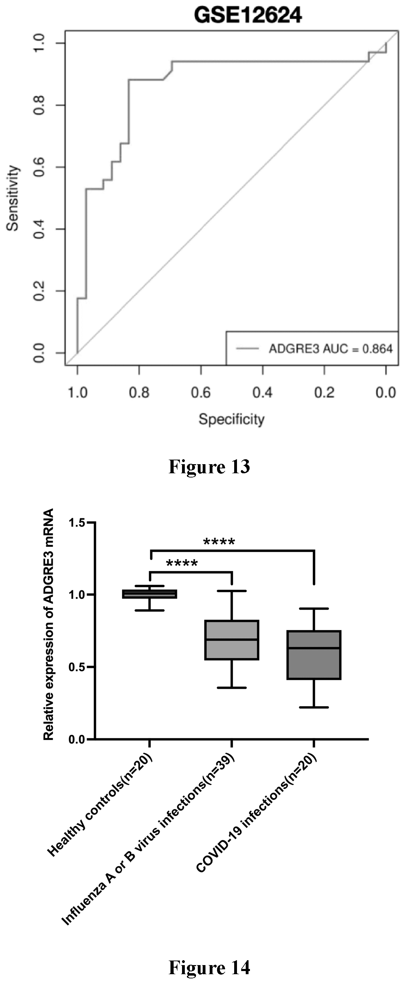

Regarding non-infectious systemic inflammation and sepsis, as shown in Figure 11, the gene expression was significantly downregulated in sepsis patients compared to healthy individuals, and significantly downregulated compared to non-infectious systemic inflammation patients. This indicates that the gene can be used as a biomarker to distinguish healthy individuals from sepsis patients (AUC = 0.920, see Figure 12) and to distinguish non-infectious systemic inflammation (SIRS) from sepsis patients (AUC = 0.864, see Figure 13).

The above results were obtained through analysis of public data. We further validated the expression of this gene or its protein level using clinical samples collected at our hospital to evaluate its utility for clinical diagnosis of systemic inflammation, treatment or prognosis of systemic inflammation, or classification of systemic inflammation.

### Example 2: Differential Expression of ADGRE3 mRNA Transcription Levels Between Healthy Individuals and Virus-Infected Patients

The transcription level of ADGRE3 mRNA in neutrophils from clinical patient samples (blood) was detected by PCR-specific amplification of ADGRE3 mRNA. The specific method is as follows:

### I. Extraction of RNA from Peripheral Blood Leukocytes

1. Centrifuge the anticoagulated blood at 4°C, 3000 rpm for 10 minutes. Transfer the serum to a 1.5 mL EP tube for storage (discard if serum is not needed).
2. Add 3 mL PBS to the anticoagulated tube, centrifuge at 4°C, 3000 rpm for 10 minutes, discard the supernatant carefully without disturbing the white buffy coat layer.
3. Prepare 1× red blood cell lysis buffer (dilution ratio 5 mL:50 mL). Add 4 mL of 1× lysis buffer to the anticoagulated tube and lyse for 15 minutes.
   (10× RBC lysis buffer formulation: 41.45 g ammonium chloride and 42.00 g sodium bicarbonate dissolved in 500 mL sterile water, pH 7.2-7.4. Note: Use ultrapure distilled water for dilution.)
4. After lysis, centrifuge at 4°C, 2500 rpm for 5 minutes, discard the supernatant. Resuspend the leukocyte pellet in 1 mL PBS and transfer to a pre-labeled 1.5 mL EP tube.
5. Centrifuge at 4°C, 2500 rpm for 5 minutes again. Discard the supernatant using a pipette and add 1 mL of TRIzol (TriQuick Reagent Total RNA Extraction Kit, Solarbio, China).
6. Add 200 µL of chloroform to the 1.5 mL EP tube, mix thoroughly by inverting and vigorously shaking for 10-15 seconds. Let stand at room temperature for 5 minutes.
7. Centrifuge at 4°C, 12,000 rpm for 15 minutes (pre-cool the centrifuge in advance).
8. Carefully aspirate the upper aqueous phase (the middle layer is a white precipitate; the bottom is a red organic phase) into a new RNase-free 1.5 mL EP tube. Add an equal volume of isopropanol, mix by inversion, and let stand at room temperature for 5 minutes.
9. Centrifuge at 4°C, 12,000 rpm for 30 minutes (pre-cool the centrifuge).
10. Discard the supernatant, add 1 mL of 75% ethanol and mix (prepare fresh; dilute anhydrous ethanol with RNase-free water).
11. Centrifuge at 4°C, 12,000 rpm for 10 minutes.
12. Discard the supernatant and air-dry the pellet at room temperature for 5-10 minutes until the white precipitate becomes transparent. Dissolve the RNA pellet in an appropriate volume (20-50 µL) of DEPC-treated water according to RNA yield.

### II. Reverse Transcription

1. Perform reverse transcription according to the instructions of the HiFiScript cDNA Synthesis Kit (CW2569M, Cwbiotech, China). Thaw all necessary reagents on ice in advance. Use 500 ng of the RNA extracted in the previous step and prepare a 20 µL reaction system according to the reverse transcription system described in Table 1.

**Table 1: Reverse Transcription Reaction System**

| **Reagent** | **Volume** |
|---|---|
| dNTP mix, 2.5 mM each | 4 µL |
| Primer mix | 2 µL |
| RNA template | 1 µL |
| 5× RT buffer | 4 µL |
| HiFi Script, 200 U/µL | 1 µL |
| RNase-Free Water | up to 20 µL |

2. Briefly centrifuge to mix the reaction components, then perform the reverse transcription reaction in a standard PCR machine under the following conditions:
42°C for 15 minutes, then 85°C for 5 minutes.
1. After the reaction is complete, remove the product, briefly centrifuge, and place it on ice for cooling.

### III. qPCR

qPCR was performed according to the instructions of the NovoStart SYBR qPCR SuperMix Plus Kit (E096-01B, GenStar, China). Template cDNA, primers, and related reagents were pre-dissolved on ice. The reaction mixture was prepared according to the following system, with a total volume of 20 µL. The qPCR reaction system is shown in Table 2:

**Table 2: qPCR Reaction System**

| **Reagent** | **Volume** |
|---|---|
| 2× Ultra SYBR mix | 10 µL |
| Forward Primer, 10 µM | 0.5 µL |
| Reverse Primer, 10 µM | 0.5 µL |
| cDNA | 9 µL |

2. Briefly centrifuge to mix the reaction components, then run the reaction on a real-time PCR instrument under the following conditions:
Initial denaturation at 95°C for 1 min, followed by 40 cycles of
   95°C for 20 s (denaturation),
   60°C for 1 min (extension).
Melting curve settings:
   95°C for 15 s,
   60°C for 1 min,
   95°C for 15 s,
   60°C for 15 s.

3. Data Analysis:
The mRNA expression levels of the target gene were calculated using the 2^-ΔΔCt method, with β-Actin or GAPDH mRNA expression as the internal control.

ADGRE3 (EMR3) qPCR primers:
Forward primer (5'→3'): **TCCAGCACGTGAAGATGACC(SEQ NO:2)
Reverse primer (5'→3'): **ACGTGTATCAGGAAGCAGCC(SEQ NO:3)

The ADGRE3 mRNA transcription levels in neutrophils were measured in:
20 healthy individuals (all clinically confirmed as normal by physical examination at our hospital), 39 patients with systemic inflammation caused by influenza A/B virus infection, and 20 patients with systemic inflammation caused by SARS-CoV-2 infection.

As shown in Figure 14, there was a highly significant difference in ADGRE3 mRNA expression in neutrophils between the virus-infected patients (influenza A/B and COVID-19) and the healthy individuals, suggesting that ADGRE3 mRNA can serve as a biomarker for diagnosing infection-induced systemic inflammation caused by viral infection.

### Exmaple 3: Differences in ADGRE3 mRNA Transcription Levels Between Healthy Individuals and Patients with Non-Infectious Systemic Inflammatory Response Syndrome (SIRS)

Patients with non-infectious systemic inflammatory response syndrome were examined, and the ADGRE3 mRNA transcription levels in leukocytes were measured and compared among the following groups:
15 healthy individuals, 27 patients with systemic inflammation caused by systemic lupus erythematosus, 25 patients with systemic inflammation caused by rheumatoid arthritis, 10 patients with systemic inflammation caused by vasculitis, and 15 patients with systemic inflammation caused by ankylosing spondylitis.

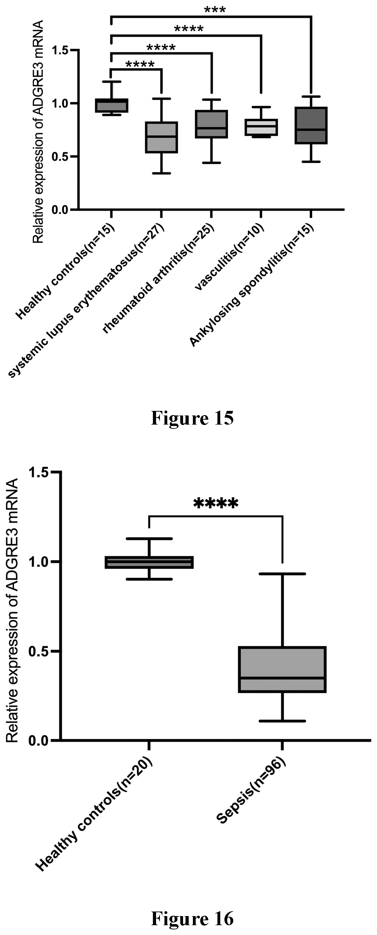

As shown in Figure 15, the leukocyte ADGRE3 mRNA transcription levels(ADGRE3 protein) in healthy individuals were significantly higher than those in all four types of autoimmune disease-induced systemic inflammation. This indicates that ADGRE3 mRNA can be used as a biomarker to distinguish healthy individuals from patients with non-infectious systemic inflammatory response syndrome.

### Example4: Differences in ADGRE3 mRNA Transcription Levels Between Healthy Individuals and Patients with Sepsis

The ADGRE3 mRNA transcription levels in leukocytes were measured and compared between 20 healthy individuals and 96 patients with sepsis (systemic inflammation). As shown in Figure 16, the ADGRE3 mRNA transcription levels in neutrophils of healthy individuals were significantly higher than those in patients with sepsis. This further confirms that ADGRE3 mRNA can serve as a biomarker to distinguish healthy individuals from sepsis patients.

### Example5: Differences in ADGRE3 Protein Expression Levels Between Healthy Individuals and Patients with Virus-Induced Systemic Inflammation

The experiments described in Examples 2 through 4 demonstrated that the transcription level of ADGRE3 mRNA in leukocytes can be used to diagnose systemic inflammation, including viral infection, non-infectious systemic inflammatory response syndrome (SIRS), and sepsis. This example further investigates the expression level of ADGRE3 protein and describes a method for detecting ADGRE3 protein expression in neutrophils as follows:
Whole peripheral blood anticoagulated with EDTA or sodium citrate containing 1×10⁶ leukocytes was collected. Then, 2 µL of fluorescein-labeled mouse anti-human ADGRE3 antibody was added, mixed thoroughly by vortexing, and incubated in the dark at room temperature for 20 minutes. After that, 2 mL of red blood cell lysis buffer was added, mixed thoroughly again, and incubated in the dark for 15 minutes. The cells were then centrifuged at 2000 rpm for 3 minutes, the supernatant was discarded, and the pellet was resuspended. After adding 2 mL of phosphate-buffered saline (PBS) or saline and centrifuging again at 2000 rpm for 3 minutes, the supernatant was removed, and the pellet was resuspended in PBS. Flow cytometry was performed using a Beckman DxFLEX instrument. During flow cytometric analysis, neutrophils, monocytes, and lymphocytes were gated separately based on FSC and SSC profiles, and the mean fluorescence intensity (MFI) of ADGRE3 in neutrophils was calculated.

### Results:

Neutrophil ADGRE3 protein levels were measured in:26 healthy individuals, 89 patients with systemic inflammation due to influenza A and B virus infection, 22 patients with systemic inflammation due to COVID-19, 5 patients with systemic inflammation due to dengue virus infection, and 5 patients with systemic inflammation due to SFTSV infection.

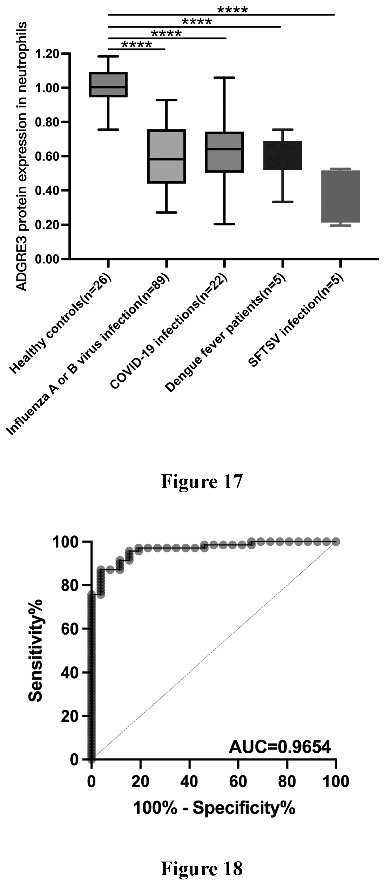

As shown in Figure 17, the neutrophil ADGRE3 protein levels in healthy individuals were significantly higher than those in all groups of virus-infected patients. In each case, the ADGRE3 protein in neutrophils decreased significantly after viral infection. These results suggest that ADGRE3 protein levels in neutrophils can distinguish between healthy individuals and patients with virus-induced systemic inflammation.

To quantify neutrophil ADGRE3 expression in healthy, non-inflammatory individuals, flow cytometry was used to calculate the mean fluorescence intensity (MFI) for each sample. The average MFI of ADGRE3 in neutrophils from healthy individuals was calculated. Under the same conditions, the MFI of ADGRE3 in patient neutrophils was also measured. The neutrophil ADGRE3 expression index (nADGRE3) was then obtained by dividing the patient's neutrophil ADGRE3 MFI by the average MFI from the healthy control group: nADGRE3 = (Patient Neutrophil ADGRE3 MFI) / (Mean Healthy Control Neutrophil ADGRE3 MFI)

By selecting different ratios of mean fluorescence intensity (MFI) as thresholds, a sample with an nADGRE3 value greater than the threshold is diagnosed as a healthy individual, whereas a value below the threshold is diagnosed as a patient with virus-induced systemic inflammation (including systemic inflammation caused by influenza A and B virus, SARS-CoV-2, dengue virus, and SFTSV infections). The sensitivity and specificity (calculated as: Sensitivity = [True Positives / (True Positives + False Negatives)] × 100%;Specificity = [True Negatives / (True Negatives + False Positives)] × 100%)are shown in Table 3. The ROC curve was plotted accordingly (Figure 18), and the AUC value was calculated to be 0.9654, indicating that ADGRE3 protein level is capable of diagnosing virus-induced systemic inflammation.

**Table 3: Neutrophil ADGRE3 Protein Expression Levels for Diagnosing Virus-Induced**

| Systemic Inflammation | | |
|---|---|---|
| **nADGRE3 Cutoff Value** | **Sensitivity (%)** | **Specificity (%)** |
| 0.7559 | 75.51 | 96.15 |
| 0.7733 | 80.00 | 96.15 |
| 0.7912 | 84.29 | 96.15 |
| 0.8072 | 87.14 | 96.15 |
| 0.8092 | 87.14 | 92.31 |
| 0.8209 | 88.57 | 88.46 |
| 0.8361 | 91.43 | 88.46 |

Based on the above results, the neutrophil ADGRE3 protein expression levels were further measured in 26 healthy individuals, 36 patients with mild virus-induced systemic inflammation, 20 patients with severe illness, and 12 critically ill patients.

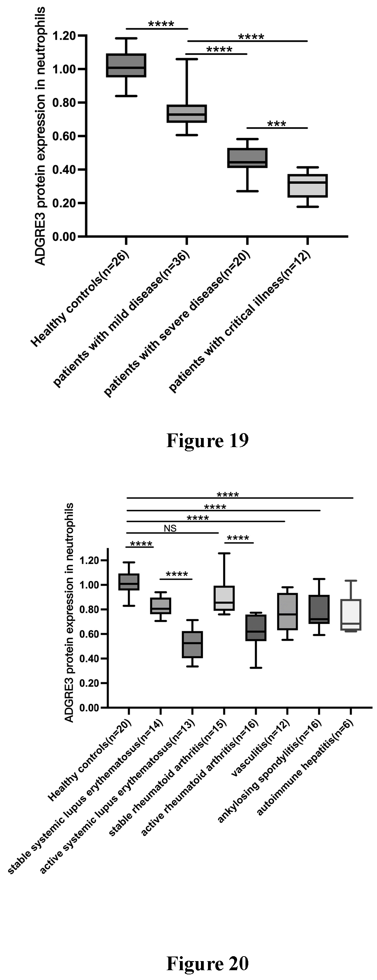

As shown in Figure 19, the neutrophil ADGRE3 protein levels showed highly significant differences between healthy individuals, mild cases, severe cases, and critical cases. As disease severity increased, ADGRE3 protein expression in neutrophils progressively decreased, indicating that ADGRE3 protein expression level can be used to stratify patients with virus-induced systemic inflammation by disease severity.

This also demonstrates that, although all these patients have systemic inflammation, the severity varies, and the ADGRE3 protein expression level disclosed in the present invention can also serve as a biomarker for stratifying the severity of systemic inflammation. This allows for timely and appropriate therapeutic interventions. Moreover, it may also be used as a biomarker for monitoring changes in disease severity, offering utility in disease surveillance and early warning.

### Example 6: Differences in ADGRE3 Protein Levels Between Healthy Individuals and Patients with Non-Infectious Systemic Inflammation

To further investigate the difference in ADGRE3 protein expression between healthy individuals and patients with non-infectious systemic inflammation, the neutrophil ADGRE3 protein expression levels were assessed in patients with autoimmune diseases. Neutrophil ADGRE3 protein expression was measured in 20 healthy individuals, 27 patients with systemic lupus erythematosus (SLE)-induced systemic inflammation (including 14 patients in the stable phase and 13 in the active phase), 31 patients with rheumatoid arthritis (RA)-induced systemic inflammation (including 15 in the stable phase and 16 in the active phase), 12 patients with vasculitis-induced systemic inflammation, 16 patients with ankylosing spondylitis-induced systemic inflammation, and 6 patients with autoimmune hepatitis-induced systemic inflammation.

As shown in Figure 20, except for stable-phase RA patients, significant differences in neutrophil ADGRE3 protein expression were observed between healthy individuals and all other groups of patients with systemic inflammation. These findings suggest that ADGRE3 protein expression may serve as a diagnostic biomarker for autoimmune diseases.

Notably, among patients with systemic inflammation, the neutrophil ADGRE3 protein expression levels were significantly different between stable-phase and active-phase patients in both SLE and RA groups, indicating that ADGRE3 protein expression can not only diagnose autoimmune diseases but also distinguish between stable and active phases of SLE and RA.

Non-infectious systemic inflammation also includes inflammation caused by transplant rejection. The neutrophil ADGRE3 protein levels were measured in 16 healthy individuals, 6 patients with kidney transplant rejection-induced systemic inflammation, and 8 patients with bone marrow transplant rejection-induced systemic inflammation.

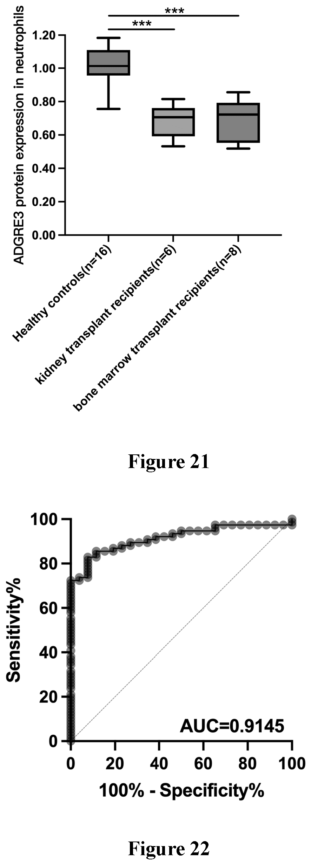

As shown in Figure 21, the neutrophil ADGRE3 protein levels in transplant rejection patients were significantly lower than those in healthy individuals, suggesting that neutrophil ADGRE3 protein expression can distinguish between healthy individuals and those with transplant rejection-induced systemic inflammation.

These findings in autoimmune disease-associated and transplant rejection-associated systemic inflammation suggest that neutrophil ADGRE3 protein may also serve as a useful indicator in other types of non-infectious systemic inflammation.

Using the same method as Example 5, the nADGRE3 value of each sample was calculated. By selecting different MFI ratio thresholds, samples with nADGRE3 values greater than the threshold were diagnosed as healthy, and those below the threshold were diagnosed as having non-infectious systemic inflammation. The sensitivity and specificity are shown in Table 4. The ROC curve was plotted accordingly (Figure 22), and the AUC was calculated to be 0.9145, demonstrating that ADGRE3 protein expression is a reliable diagnostic biomarker for non-infectious systemic inflammation.

**Table 4: Neutrophil ADGRE3 Protein Expression and Diagnosis of Non-Infectious Systemic Inflammation**

| **nADGRE3 Cutoff Value** | **Sensitivity (%)** | **Specificity (%)** |
|---|---|---|
| 0.8363 | 73.68 | 96.15 |
| 0.8555 | 75.00 | 92.31 |
| 0.8887 | 78.95 | 92.31 |
| 0.8983 | 81.58 | 92.31 |
| 0.9129 | 82.89 | 92.31 |
| 0.9362 | 84.21 | 88.46 |
| 0.9429 | 85.53 | 84.62 |

### Example 7: Differences in ADGRE3 Protein Expression Levels Between Healthy Individuals and Sepsis Patients

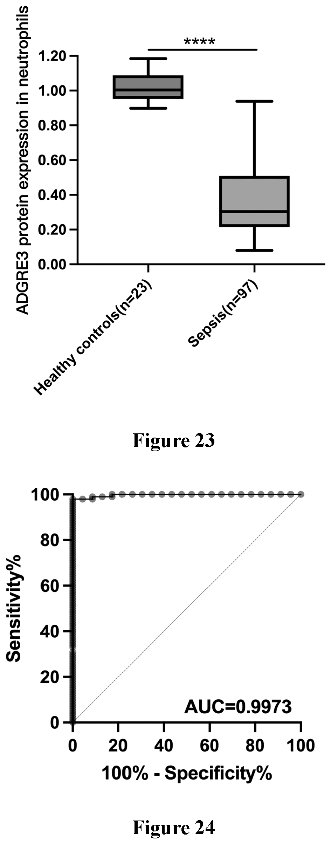

The expression level of ADGRE3 protein in neutrophils was analyzed in patients with sepsis. As shown in Figure 23, the neutrophil ADGRE3 protein levels of healthy individuals (n = 23) and sepsis patients (n = 97) exhibited a highly significant difference, suggesting that ADGRE3 can be used as a biomarker for the diagnosis of sepsis.

Using the same method as Example 5, nADGRE3 values were calculated for each sample. Different thresholds based on the ratio of mean fluorescence intensities (MFI) were applied. Samples with nADGRE3 values greater than the threshold were diagnosed as healthy, and those below the threshold were diagnosed as having sepsis. The sensitivity and specificity at each cutoff are listed in Table 5. Based on these data, an ROC curve was plotted (Figure 24), and the AUC was calculated to be 0.9973, indicating that ADGRE3 protein expression is a highly accurate diagnostic biomarker for sepsis.

**Table 5: Neutrophil ADGRE3 Protein Expression and Sepsis Diagnosis**

| **nADGRE3 Cutoff Value** | **Sensitivity (%)** | **Specificity (%)** |
|---|---|---|
| 0.7509 | 93.81 | 100.00 |
| 0.7731 | 94.85 | 100.00 |
| 0.8990 | 97.94 | 95.65 |
| 0.9050 | 98.97 | 91.30 |

To assess the diagnostic value of ADGRE3 in different types of sepsis, neutrophil ADGRE3 expression was measured in 23 healthy individuals, 33 patients with Gram-positive bacterial sepsis, 44 patients with Gram-negative bacterial sepsis, and 10 patients with fungal sepsis.

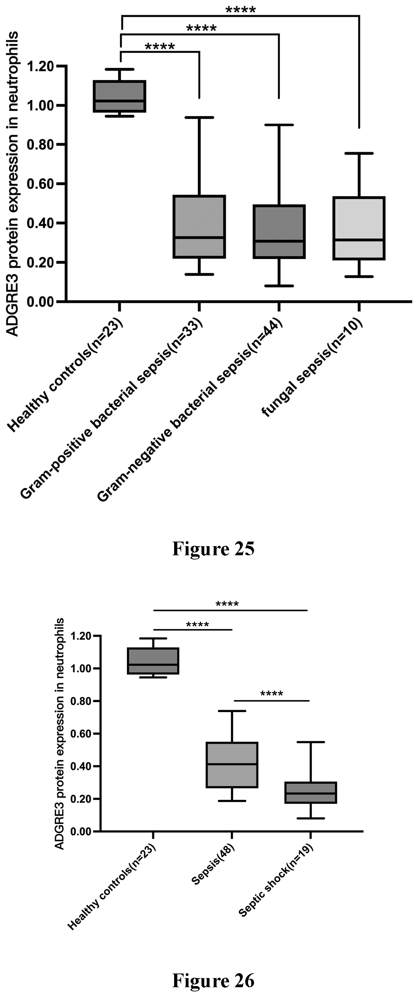

As shown in Figure 25, ADGRE3 protein levels in neutrophils were significantly different between healthy individuals and each of the three sepsis subgroups, suggesting that ADGRE3 is capable of diagnosing multiple types of sepsis.

To investigate the correlation between ADGRE3 expression and sepsis severity, neutrophil ADGRE3 protein levels were measured in 23 healthy individuals, 48 sepsis patients, and 19 patients with septic shock.

Figure 26 shows that neutrophil ADGRE3 levels were significantly different across the three groups and decreased with increasing severity, indicating that ADGRE3 levels may be used to stratify sepsis patients by disease severity.

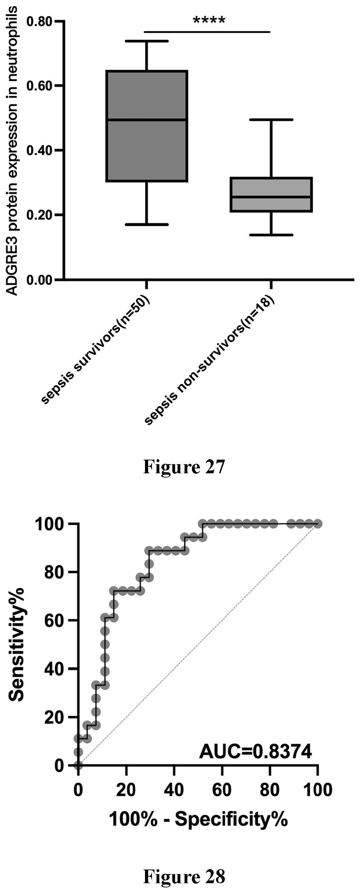

A retrospective analysis was also performed comparing surviving (n = 50) and deceased (n = 18) sepsis patients in terms of their neutrophil ADGRE3 expression levels during the disease. As shown in Figure 27, there was a highly significant difference between survivors and non-survivors, suggesting that ADGRE3 protein expression may be used to predict the risk of death in sepsis patients.

Following the method of Example 5, nADGRE3 values were again calculated. Using different threshold values, a low nADGRE3 predicted high mortality risk, while a high nADGRE3 predicted low risk. The predictive performance was compared to actual outcomes (survival or death), and sensitivity and specificity values are presented in Table 6. The corresponding ROC curve is shown in Figure 28, and the AUC was 0.8374, demonstrating that ADGRE3 protein expression has predictive value for mortality risk in sepsis.

**Table 6: Neutrophil ADGRE3 Protein Expression and Mortality Risk Prediction in Sepsis Patients**

| **nADGRE3 Cutoff Value** | **Sensitivity (%)** | **Specificity (%)** |
|---|---|---|
| 0.2596 | 61.11 | 88.89 |
| 0.2822 | 72.22 | 85.19 |
| 0.2943 | 72.22 | 74.07 |
| 0.3185 | 77.78 | 74.07 |
| 0.3605 | 83.33 | 70.37 |
| 0.4570 | 88.89 | 59.26 |

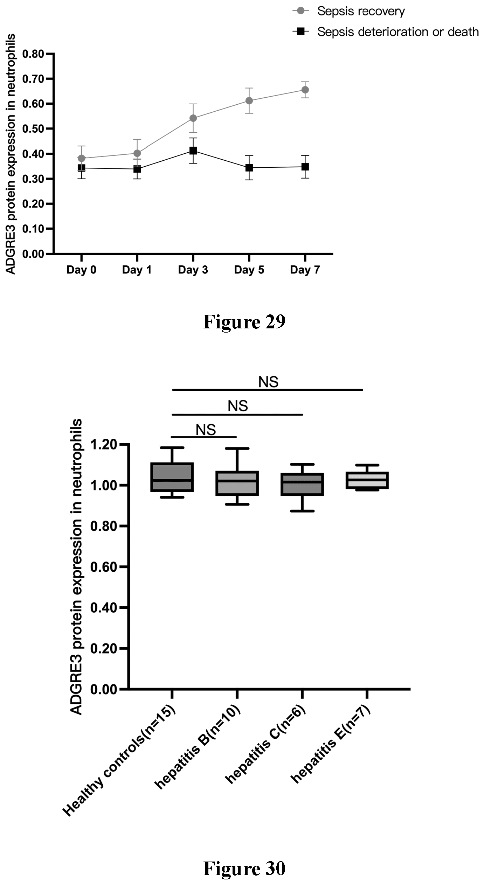

A retrospective analysis was also conducted to examine whether neutrophil ADGRE3 expression could predict improvement in sepsis. Sepsis patients were grouped into those who improved and those who either did not improve by Day 7 or died. ADGRE3 protein levels were tracked daily from Day 0 (diagnosis). As shown in Figure 29, patients who showed improvement had consistently higher ADGRE3 expression levels than those who did not improve or died. In the improving group, ADGRE3 levels increased markedly from Day 3 to Day 5, while in the non-improving or deceased group, ADGRE3 expression declined during this period. These findings suggest that ADGRE3 protein expression can predict sepsis prognosis, especially when monitoring changes from Day 3 to Day 5, providing a valuable early indicator of clinical improvement or deterioration.

### Example 8: No Significant Difference in ADGRE3 Protein Expression in Neutrophils Between Healthy Individuals and Patients with Localized (Non-Systemic) Inflammation

In Example 1, analysis of public datasets revealed that ADGRE3 gene expression has high specificity for diagnosing systemic inflammation. Therefore, the ADGRE3 protein levels in healthy individuals and patients with localized inflammation should not show significant differences.

To verify this, neutrophil ADGRE3 protein expression levels were measured in 15 healthy individuals, and localized inflammation patients, including: 10 patients with hepatitis B, 6 patients with hepatitis C, and 7 patients with hepatitis E.

As shown in Figure 30, there were no significant differences in neutrophil ADGRE3 protein expression levels between healthy individuals and patients with localized inflammation.

This result indicates that neutrophil ADGRE3 protein expression can be used to diagnose systemic inflammation without interference from localized inflammation, confirming its high specificity.

### Example 9: Application of Neutrophil ADGRE3 Protein Combined with CD64, PCT, and CRP in Differentiating Types of Inflammation

PCT (procalcitonin), CRP (C-reactive protein), IL-6, and the neutrophil CD64 infection index (nCD64) are currently used clinical biomarkers for bacterial infection and systemic inflammatory response.

In this study, flow cytometry was used to detect mean fluorescence intensity (MFI) of neutrophil ADGRE3 in peripheral blood samples from: Healthy controls (n = 42), Patients with systemic inflammatory response of known infectious origin (including viral, bacterial, and fungal infections, n = 218), Patients with autoimmune diseases (n = 92), and patients experiencing transplant rejection (n = 14).

The relative expression level of neutrophil ADGRE3 (nADGRE3) in systemic inflammation patients was calculated as:nADGRE3 = (MFI of neutrophil ADGRE3 in patient) / (MFI of neutrophil ADGRE3 in healthy controls)

Statistical analysis determined the optimal nADGRE3 cutoff value as 0.8361; a value of nADGRE3 < 0.8907 was interpreted as indicating a systemic inflammatory response.

Simultaneously, serum data for PCT, CRP, IL-6, and nCD64 infection index were collected and analyzed. The performance of nADGRE3, nCD64, PCT, CRP, and IL-6 was compared across three inflammatory types:
Virus-induced systemic inflammatory response, Sepsis, and Non-infectious systemic inflammation (including autoimmune diseases and transplant rejection). Results are summarized in Table 7.

**Table 7: Application of Neutrophil nADGRE3 Combined with Other Biomarkers in Differentiating Inflammation Types**

| **Inflammation Type** | **nADGRE3** | **nCD64** | **PCT (Procalcitonin)** | **CRP (C-Reactive Protein)** | **IL-6** |
|---|---|---|---|---|---|
| **Virus-induced systemic inflammation (n = 121)** | Decrease n= 107 (88.42%) | Increase n=16 (13.22%) | Increase n=3 (2.48%) | Increase n= 56 (46.28%) | Increase n=73 (60.33%) |
| **Sepsis (n = 97)** | Decrease n= 95 (97.93%) | Increase n=75 (77.31%) | Increase n=89 (91.75%) | Increase n=85 (87.62%) | Increase n= 96 (98.96%) |
| **Non-infectious systemic inflammation (e.g., autoimmune diseases and transplant rejection, n = 106)** | Decrease n= 84 (79.24%) | Increase n=14 (13.20%) | Increase n=2 (1.88%) | Increase n=57 (53.77%) | Increase n=46 (43.39%) |

A comparison between neutrophil nADGRE3 and existing clinical inflammatory markers revealed that nADGRE3 exhibits extremely high sensitivity for indicating systemic inflammation. A larger proportion of systemic inflammation patients showed decreased neutrophil nADGRE3 levels. Therefore, more systemic inflammation patients can be diagnosed based on laboratory detection of reduced neutrophil nADGRE3.

In contrast, the sensitivity of existing clinical inflammatory markers is low in systemic inflammation caused by viral infections and non-infectious systemic inflammation patients, which may lead to normal test results and subsequent misdiagnosis. Thus, neutrophil nADGRE3 demonstrates higher sensitivity for indicating systemic inflammation compared to currently used clinical markers, indicating that nADGRE3 has high sensitivity in diagnosing systemic inflammation, resulting in higher diagnostic accuracy.

At the same time, when diagnosing systemic inflammation, current clinical inflammatory markers lack specificity and cannot exclude interference from localized inflammation. As a result, diagnosis often requires integration with imaging techniques. For example, patients with multiple localized inflammatory sites, commonly seen in autoimmune diseases, may not actually have systemic inflammation. If imaging suggests multiple inflammatory sites and current clinical inflammatory markers are considered, there is a high risk of misclassifying these patients as having systemic inflammation.

By contrast, neutrophil nADGRE3 has sufficiently high specificity and can be used for diagnosis without relying on imaging. When neutrophil nADGRE3 decreases, the patient can be specifically diagnosed with systemic inflammation, effectively excluding the interference of localized inflammation. Therefore, neutrophil nADGRE3 has higher specificity than current clinical inflammatory markers for diagnosing systemic inflammation, leading to higher diagnostic accuracy.

Systemic inflammation patients were classified by etiology into bacterial infection-induced systemic inflammatory response patients, viral infection-induced systemic inflammatory response patients, and systemic inflammatory response patients caused by autoimmune diseases and transplant rejection. These groups were categorized according to nADGRE3 and other inflammatory markers (CRP, PCT, IL-6, nCD64), as shown in Figures 31, 32, and 33, respectively.

Figure 31 shows that among patients with systemic inflammatory response caused by bacterial infection, 96.51% exhibited decreased ADGRE3 protein expression levels. Among those with decreased ADGRE3 protein expression, the proportions of patients with elevated markers were as following: CRP 92.86%, PCT 93.02%, IL-6 96.42%, and nCD64 98.81%.

Figure 32 shows that among patients with systemic inflammatory response caused by viral infection (a type of non-bacterial systemic inflammation), 88.42% had decreased ADGRE3 protein expression. Among these patients, the proportions with elevated markers were as following: CRP 48.59%, PCT 0.93% (only 1 case), IL-6 64.48%, and nCD64 8.41% (only 9 cases).

Figure 33 shows that among patients with systemic inflammatory response caused by autoimmune diseases and transplant rejection (also a type of non-bacterial systemic inflammation), 79.25% had decreased ADGRE3 protein expression. Some patients taking immunosuppressants or steroids exhibited increased ADGRE3 protein expression and were classified as normal. Among those with decreased ADGRE3 expression, the proportions with elevated markers were as following: CRP 61.90%, PCT 1.19% (only 1 case), IL-6 53.57%, and nCD64 8.33% (only 7 cases).

In patients with systemic inflammatory responses caused by viral infection or autoimmune diseases and transplant rejection, the proportion of patients with decreased ADGRE3 expression who also had elevated PCT and nCD64 was very low. This is because PCT and nCD64 are markers specific for diagnosing bacterial inflammation, having specificity for bacterial systemic inflammation diagnosis. Cases with elevated PCT and nCD64 were accompanied by bacterial infections. In addition, other markers for diagnosing bacterial inflammation include HBP (heparin-binding protein), among others.

Therefore, the neutrophil ADGRE3 protein expression level can be combined with markers used to diagnose bacterial inflammation to classify or localize systemic inflammation patients, distinguishing bacterial systemic inflammation from non-bacterial systemic inflammation. Specifically, by detecting ADGRE3 protein expression levels alongside bacterial inflammation markers, if ADGRE3 protein expression is decreased and the bacterial inflammation markers are altered, the diagnosis is bacterial systemic inflammation; if ADGRE3 protein expression is decreased but the bacterial inflammation markers are within normal ranges, the diagnosis is non-bacterial systemic inflammation.

An "alteration" in the bacterial inflammation marker levels means the marker's detected value falls outside the normal range observed in healthy individuals, either elevated or decreased, both indicating bacterial inflammation; "normal" marker levels mean the detected value is within the normal range, indicating no bacterial inflammation.

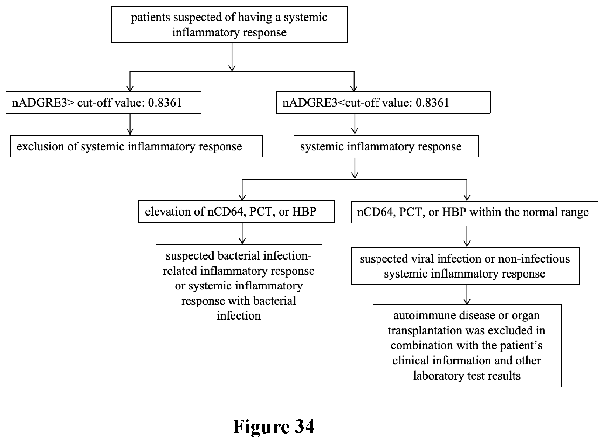

Figure 34 is a flowchart for diagnosing suspected systemic inflammatory response patients to determine whether they have systemic inflammation and to classify systemic inflammation patients into bacterial or non-bacterial systemic inflammation. First, the neutrophil nADGRE3 level is measured and compared to a threshold of 0.8361; values above this threshold exclude systemic inflammatory response, while values below diagnose systemic inflammation. Then, for confirmed systemic inflammation patients, markers such as nCD64, PCT, and HBP, used to diagnose bacterial systemic inflammation, are tested. If these markers are elevated, bacterial infection-induced systemic inflammation or bacterial co-infection is considered. If these markers are normal, viral infection or non-infectious systemic inflammation is considered, and further confirmation can be made using clinical information and other laboratory tests. For example, if the patient has a history of organ transplantation, systemic inflammation caused by transplant rejection may be considered.

All patents and publications mentioned in this specification represent publicly available technologies in the field and can be used in the present invention. All patents and publications cited here are also listed as references and are individually incorporated by reference to the same extent as if each were individually cited. The present invention can be realized with the absence of one or more elements or limitations unless specifically stated otherwise. For example, in each embodiment, the terms "comprising," "consisting essentially of," and "consisting of" may be used interchangeably. The term "one" as used herein merely means "single" and does not exclude the presence of multiple; it may also mean "one or more." The terminology and expressions used are for descriptive purposes only and are not intended to limit the scope. No intent is made to exclude any equivalents of the features described herein. It is understood that any suitable modifications or changes can be made within the scope of the invention and claims. The embodiments described are preferred examples and features, and those skilled in the art can make various changes based on the essence of the invention described herein. Such changes are considered within the scope of the invention and the independent and dependent claims.

## Claims

1. A method for diagnosing whether an individual is suffering from systemic inflammation comprising:
obtaining a biological sample from an individual;
detecting a level of a biomarker in the biological sample;
comparing the detected level of the biomarker with a predetermined threshold; and
if the detected level of the biomarker is higher than or equal to the predetermined threshold, determining that the individual is not suffered from systemic inflammation;
if the detected level of the biomarker is less than the predetermined threshold, determining that the individual is suffered from systemic inflammation;
**characterized in that**:
the biomarker comprising an ADGRE3 protein; an ADGRE3 protein fragment; an amino acid sequence corresponding to ADGRE3 or peptide fragment of an ADGRE3 protein; ADGRE3 mRNA or an ADGRE3 mRNA fragment.

2. The method according to claim 1, **characterized in that**, an ADGRE3 protein; an ADGRE3 protein fragment; an amino acid sequence corresponding to ADGRE3 or peptide fragment of an ADGRE3 protein; ADGRE3 mRNA or an ADGRE3 mRNA fragment is included on/in neutrophils or monocytes that be included in the sample.

3. The method according to claim 1, **characterized in that**, the biological sample is selected one or more of below sample: peripheral blood, urine, secretions, pus, body fluid, cerebrospinal fluid, or fresh tissue.

4. The method according to claim 1, **characterized in that**, the systemic inflammation comprises infectious systemic inflammation or non-infectious systemic inflammation.

5. The method according to claim 4, **characterized in that**, the infectious systemic inflammation comprises bacterial infection, viral infection, fungal infection, or parasitic infection.

6. The method according to claim 5, **characterized in that**, the viral infection comprises influenza virus infection, coronavirus infection, respiratory syncytial virus infection, dengue virus infection, or novel bunyavirus infection.

7. The method according to claim 4, **characterized in that**, the non-infectious systemic inflammation comprised autoimmune disease, transplant rejection, inflammatory bowel disease, allergic reaction, metabolic disease, or tissue injury.

8. The method according to claim 7, **characterized in that**, the autoimmune disease comprises systemic lupus erythematosus, rheumatoid arthritis, vasculitis, ankylosing spondylitis, or autoimmune hepatitis.

9. The method according to claim 7, **characterized in that**, the transplant rejection comprises bone marrow transplant rejection, kidney transplant rejection, or liver transplant rejection.

10. The method according to claim 1, **characterized in that**, the systemic inflammation comprises sepsis.

11. The method according to claim 10, **characterized in that**, the sepsis is associated with one or more according to Gram-positive bacterial infection, Gram-negative bacterial infection, or fungal infection.

12. The method according to claim 10, **characterized in that**, the sepsis comprises septic shock.

13. The method according to any one of claims 1 to 12, **characterized in that** the predetermined threshold is a level or concentration of the ADGRE3 protein; the ADGRE3 protein fragment; the amino acid sequence corresponding to the ADGRE3 or the peptide fragment of the ADGRE3 protein; the ADGRE3 mRNA or the ADGRE3 mRNA fragment in a healthy population.

14. A method for classifying a patient with systemic inflammation caused by viral infection into a mild, severe, or critical condition, the method comprising:
obtaining a biological sample from the patient;
detecting a level of a biomarker in the biological sample;
comparing the detected level with a predetermined threshold; and
**characterized in that**.
the biomarker comprises:
an ADGRE3 protein; an ADGRE3 protein fragment; an amino acid sequence corresponding to ADGRE3 or peptide fragment of an ADGRE3 protein; ADGRE3 mRNA or an ADGRE3 mRNA fragment.

15. The method according to claim 14, **characterized in that**, the predetermined threshold is a level or concentration of the ADGRE3 protein; the ADGRE3 protein fragment; the amino acid sequence corresponding to the ADGRE3 or the peptide fragment of the ADGRE3 protein in a sample of a critical condition of a patient, if the detected level is higher than the predetermined threshold, the patient is classified into severe condition.

16. The method according to claim 14, **characterized in that**, the predetermined threshold is a level or concentration of ADGRE3 protein ; a fragment thereof; an amino acid sequence or peptide fragment of ADGRE3, in a sample from a patient with severe systemic inflammation caused by viral infection, and **characterized in that** the patient is classified into the critical systemic inflammation if the detected level is less than the predetermined threshold.

17. The method according to claim 14, **characterized in that** the predetermined threshold comprises a first threshold and a second threshold, **characterized in that**:
the first threshold is a level or concentration of ADGRE3 protein; a fragment thereof ;an amino acid sequence or peptide fragment of ADGRE3, in a sample from a patient with mild systemic inflammation caused by viral infection; and the second threshold corresponds is a level or concentration of ADGRE3 protein; a fragment thereof ;an amino acid sequence or peptide fragment of ADGRE3 in sample from a patient with critical systemic inflammation caused by viral infection; and **characterized in that** the patient is classified as having severe systemic inflammation caused by viral infection if the detected level is less than the first threshold and greater than the second threshold.

18. A method for classifying a patient with systemic inflammation caused by systemic lupus erythematosus (SLE) as being in a stable phase or active phase, the method comprising:
obtaining a biological sample from the patient;
detecting a level of a biomarker in the sample;
comparing the detected level with a predetermined threshold;
if the detected level of the biomarker is higher than or equal to the predetermined threshold, determining that the patent is in a stable phase; if the detected level of the biomarker is less than the predetermined threshold, determining that patent is in the active phase; and
**characterized in that**.
the biomarker comprises:
an ADGRE3 protein; an ADGRE3 protein fragment; an amino acid sequence corresponding to ADGRE3 or peptide fragment of an ADGRE3 protein; ADGRE3 mRNA or an ADGRE3 mRNA fragment.

19. The method according to claim 18, **characterized in that** the threshold is a level or concentration of an ADGRE3 protein; an ADGRE3 protein fragment; an amino acid sequence corresponding to ADGRE3 or peptide fragment of an ADGRE3 protein in a sample from patients with SLE in the stable phase.

20. A method for classifying a patient with systemic inflammation caused by rheumatoid arthritis (RA) as being in a stable phase or active phase, the method comprising:
obtaining a biological sample from the patient;
detecting a level of a biomarker in the sample;
comparing the detected level with a predetermined threshold;
if the detected level of the biomarker is higher than or equal to the predetermined threshold, determining that the patent is in a stable phase; if the detected level of the biomarker is less than the predetermined threshold, determining that patent is in the active phase; and **characterized in that**.
the biomarker comprises:
an ADGRE3 protein; an ADGRE3 protein fragment; an amino acid sequence corresponding to ADGRE3 or peptide fragment of an ADGRE3 protein; ADGRE3 mRNA or an ADGRE3 mRNA fragment.

21. The method according to claim 20, **characterized in that** the threshold is a level or concentration of an ADGRE3 protein; an ADGRE3 protein fragment; an amino acid sequence corresponding to ADGRE3 or peptide fragment of an ADGRE3 proteinr in samples from RA patients in the stable phase.

22. A method for predicting a mortality risk of a patient with sepsis, the method comprising:
obtaining a biological sample from the patient;
detecting a level of a biomarker in the sample;
comparing the detected level with a predetermined threshold;
if the detected level of the biomarker is higher than or equal to the predetermined threshold, determining that the mortality risk of the patent is low; if the detected level of the biomarker is less than the predetermined threshold, determining that the mortality risk of the patent is increased or high; and
**characterized in that**.
the biomarker comprises:
an ADGRE3 protein; an ADGRE3 protein fragment; an amino acid sequence corresponding to ADGRE3 or peptide fragment of an ADGRE3 protein; ADGRE3 mRNA or an ADGRE3 mRNA fragment.

23. The method according to claim 22, **characterized in that** the threshold is a level or concentration of an ADGRE3 protein; an ADGRE3 protein fragment; an amino acid sequence corresponding to ADGRE3 or peptide fragment of an ADGRE3 protein in a sample from sepsis patients with low mortality risk.

24. The method according to claim 22, further comprising:
if a level of ADGRE3 protein or a fragment thereof; a corresponding amino acid sequence or peptide fragment in a sample collected from the patient on the fifth day after the patient is diagnosed as a sepsis patient is higher than a level of ADGRE3 protein or a fragment thereof; a corresponding amino acid sequence or peptide fragment in a sample collected from the patient on the third day after the patient is diagnosed as a sepsis patient; predicting that the sepsis condition is improving, if not, predicting that the sepsis condition is not improving or will be death.

25. A method for diagnosing whether an individual has bacterial systemic inflammation, the method comprising:
obtaining a biological sample from the patient;
detecting a level of a biomarker in the sample;
comparing the detected level with a predetermined threshold;
**characterized in that**.
the biomarker comprises: an ADGRE3 protein; an ADGRE3 protein fragment; an amino acid sequence corresponding to ADGRE3 or peptide fragment of an ADGRE3 protein; ADGRE3 mRNA or an ADGRE3 mRNA fragment.

26. The method according to claim 25, **characterized in that** the threshold is a level or concentration of an ADGRE3 protein; an ADGRE3 protein fragment; an amino acid sequence corresponding to ADGRE3 or peptide fragment of an ADGRE3 protein in a sample from a healthy population.

27. The method according to claim 25, **characterized in that** the individual is diagnosed as not having bacterial systemic inflammation if the detected level is greater than or equal to the threshold.

28. The method according to claim 25, **characterized in that**, if the detected level of the biomarkers is less than the threshold, and one or more further biomarkers specific for the diagnosis of bacterial inflammation are detected in the sample; and
if the level of the specific bacterial inflammation biomarker is outside the normal range of a healthy individual, the individual is diagnosed as having bacterial systemic inflammation; and
if the level of the specific bacterial inflammation biomarker is within the normal range of a healthy individual, the individual is diagnosed as not having bacterial systemic inflammation.

29. The method according to claim 28, **characterized in that** the one or more futher biomarkers specific for bacterial inflammation are selected from the group consisting of CD64, procalcitonin (PCT), and heparin-binding protein (HBP).

30. The method according to any of claims 1 to 29, **characterized in that** the ADGRE3 protein comprises the amino acid sequence set forth in SEQ ID NO:1, or is encoded by a transcript selected from the group consisting according to XM\_054322398.1, XM\_047439546.1, XM\_011528374.3, NM\_001289158.2, NM\_001289159.2, and NM\_032571.5.

31. The method according to any one of according to claims 1 to 30, **characterized in that** the level of the ADGRE3 protein or a fragment thereof, or an amino acid sequence or peptide fragment ofADGRE3, in the sample is detected using a method comprising using an antibody to specifically bind the ADGRE3 protein or a fragment thereof in the sample.

32. The method according to claim 31, **characterized in that** the method comprising flow cytometry.

33. Use of a biomarker for the preparation of a reagent for diagnosing systemic inflammation, **characterized in that** the biomarker is selected from the group consisting of ADGRE3 protein or a fragment thereof; an amino acid sequence or peptide fragment of ADGRE3; andADGRE3 mRNA or a fragment thereof.

34. The use according to claim 33, **characterized in that** the diagnosis according to systemic inflammation comprises one or more according to :
(a) diagnosing whether an individual has systemic inflammation;
(b) assessing the risk according to developing systemic inflammation;
(c) monitoring the disease course in patients with systemic inflammation;
(d) prognostic evaluation according to patients with systemic inflammation; or
(e) guiding therapeutic treatment in systemic inflammation.

35. The use according to claim 33, **characterized in that** the reagent for diagnosing systemic inflammation is configured to detect ADGRE3 protein or a fragment thereof to in a sample from an individual, preferably ADGRE3 protein or a fragment thereof present on or in neutrophils or monocytes in the sample.

36. The use according to claim 35, **characterized in that** the sample comprises one or more of peripheral blood, urine, secretions, pus, body fluids, cerebrospinal fluid, and fresh tissue.

37. The use according to claim 33, **characterized in that** the systemic inflammation comprises infectious or non-infectious systemic inflammation.

38. The use according to claim 37, **characterized in that** the infectious systemic inflammation comprises one or more of bacterial infection, viral infection, fungal infection, and parasitic infection.

39. The use according to claim 38, **characterized in that** the viral infection comprises one or more of influenza virus infection, coronavirus infection, dengue virus infection, novel bunyavirus infection, respiratory syncytial virus infection, cytomegalovirus infection, and Epstein-Barr virus (EBV) infection.

40. The use according to claim 37, **characterized in that** the non-infectious systemic inflammation comprises one or more of autoimmune diseases, transplant rejection, inflammatory bowel disease, allergic reactions, metabolic disorders, and inflammation caused by tissue injury.

41. The use according to claim 40, **characterized in that** the autoimmune diseases comprise one or more of systemic lupus erythematosus, rheumatoid arthritis, vasculitis, ankylosing spondylitis, and autoimmune hepatitis.

42. The use according to claim 41, **characterized in that** the transplant rejection-induced inflammation is caused by one or more of bone marrow transplantation, kidney transplantation, and liver transplantation.

43. The use according to claim 33, **characterized in that** the systemic inflammation comprises sepsis.

44. The use according to claim 43, **characterized in that** the sepsis is caused by one or more infections selected from pulmonary infection, biliary tract infection, urinary tract infection, gastrointestinal infection, bloodstream infection, reproductive tract infection, intra-abdominal infection, central nervous system infection, skin and tissue infection, and pyogenic osteomyelitis.

45. The use according to claim 43, **characterized in that** the sepsis comprises one or more of Gram-positive bacterial sepsis, Gram-negative bacterial sepsis, fungal sepsis, and viral sepsis.

46. The use according to claim 43, **characterized in that** the sepsis comprises septic shock.

47. Use of a biomarker for the preparation according to a reagent for distinguishing whether an individual is healthy or has systemic inflammation caused by viral infection, **characterized in that** the biomarker comprises ADGRE3 protein or a fragment thereof, or an amino acid sequence or peptide fragment corresponding to ADGRE3; or ADGRE3 mRNA or a fragment thereof.

48. The use according to claim 47, **characterized in that** the reagent is further configured to distinguish whether the individual with systemic inflammation caused by viral infection is a mild case, severe case, or critically ill case.

49. Use of a biomarker for the preparation according to a reagent for distinguishing whether an individual is healthy or has systemic inflammation caused by systemic lupus erythematosus (SLE), **characterized in that** the biomarker comprises ADGRE3 protein or a fragment thereof , or an amino acid sequence or peptide fragment of ADGRE3.

50. Use of a biomarker for the preparation according to a reagent for distinguishing whether a patient with systemic lupus erythematosus (SLE) and systemic inflammation is in a stable phase or an active phase, **characterized in that** the biomarker comprises ADGRE3 protein or a fragment thereof , or an amino acid sequence or peptide fragment corresponding to ADGRE3.

51. Use of a biomarker for the preparation according to a reagent for distinguishing whether an individual is healthy or has systemic inflammation caused by rheumatoid arthritis, **characterized in that** the biomarker comprises ADGRE3 protein or a fragment thereof, or an amino acid sequence or peptide fragment corresponding to ADGRE3; or ADGRE3 mRNA or a fragment thereof.

52. Use of a biomarker for the preparation according to a reagent for distinguishing whether a patient with systemic inflammation caused by rheumatoid arthritis is in a stable phase or an active phase, **characterized in that** the biomarker comprises ADGRE3 protein or a fragment thereof , or an amino acid sequence or peptide fragment corresponding to ADGRE3; or ADGRE3 mRNA or a fragment thereof

53. Use of a biomarker for the preparation according to a reagent for distinguishing whether an individual is healthy or has sepsis, **characterized in that** the biomarker comprises ADGRE3 protein or a fragment thereof, or an amino acid sequence or peptide fragment corresponding to ADGRE3; or ADGRE3 mRNA or a fragment thereof.

54. Use of a biomarker for the preparation according to a reagent for distinguishing whether a sepsis patient has common sepsis or septic shock, **characterized in that** the biomarker comprises ADGRE3 protein or a fragment thereof , or an amino acid sequence or peptide fragment corresponding to ADGRE3; or ADGRE3 mRNA or a fragment thereof.

55. Use of a biomarker for the preparation according to a reagent for predicting the mortality risk according to a sepsis patient, **characterized in that** the biomarker comprises ADGRE3 protein or a fragment thereof, or an amino acid sequence or peptide fragment corresponding to ADGRE3; or ADGRE3 mRNA or a fragment thereof.

56. The use according to claim 55, **characterized in that** the use further comprises: if the level of ADGRE3 protein in the sample collected on Day 5 after diagnosis is higher than that in the sample collected on Day 3, the sepsis condition of the individual is predicted to be improving; otherwise, the sepsis condition is predicted to not be improving or to be progressing toward death.

57. Use of a biomarker combination for the preparation of a reagent for diagnosing whether a systemic inflammation patient is suffering from systemic inflammation caused by bacterial infection, **characterized in that** the biomarker combination comprises ADGRE3 protein or a fragment thereof, or an amino acid sequence or peptide fragment corresponding to ADGRE3, and further comprises one or more markers specifically diagnostic of bacterial inflammation.

58. The use according to claim 57, **characterized in that** the marker specifically diagnostic of bacterial inflammation comprises one or more according to CD64, PCT, and HBP.

59. The use according to claim 57, **characterized in that** if the level of ADGRE3 protein or a fragment thereof , or the corresponding amino acid sequence or peptide fragment in the sample is below a preset threshold, and the level according to the marker specifically diagnostic according to bacterial inflammation in the sample falls outside the range observed in healthy individuals, the patient is determined to have systemic inflammation caused by bacterial infection.

60. The use according to claim 57, **characterized in that** if the level according to ADGRE3 protein or a fragment thereof , or the corresponding amino acid sequence or peptide fragment in the sample is below a preset threshold, and the level according to the marker specifically diagnostic according to bacterial inflammation in the sample falls within the range observed in healthy individuals, the patient is determined to have systemic inflammation caused by viral infection or non-infectious disease.

61. The use according to claim 59 or 60, **characterized in that** the threshold is the level or content of the ADGRE3 protein or a fragment thereof, or the corresponding amino acid sequence or peptide fragment in the samples from healthy individuals.

62. Use of a biomarker for the preparation of a reagent for distinguishing whether an individual has systemic inflammation caused by transplant rejection, **characterized in that** the biomarker comprises ADGRE3 protein or a fragment thereof , or an amino acid sequence or peptide fragment corresponding to ADGRE3; or ADGRE3 mRNA or a fragment thereof.

63. The method or use according to any one of claims 1 to 62, **characterized in that** the ADGRE3 protein has the amino acid sequence as shown in SEQ ID NO:1; or the ADGRE3 protein is translated from any one of the mRNA sequences: XM\_054322398.1, XM\_047439546.1, XM\_011528374.3, NM\_001289158.2, NM\_001289159.2, or NM\_032571.5.

64. The method or use according to any one of claims 1 to 62, **characterized in that** the ADGRE3 mRNA comprises any one or more according to the nucleotide sequences shown in XM\_054322398.1, XM\_047439546.1, XM\_011528374.3, NM\_001289158.2, NM\_001289159.2, or NM\_032571.5.
